(19) Europäisches Patentamt
European Patent Office
Office européen des brevets

(11) EP 2 324 047 B1

# (12) EUROPEAN PATENT SPECIFICATION

(45) Date of publication and mention of the grant of the patent:
**25.02.2015 Bulletin 2015/09**

(21) Application number: **08787073.9**

(22) Date of filing: **08.08.2008**

(51) Int Cl.:
***C07K 7/56*** *(2006.01)* ***C07K 7/64*** *(2006.01)*
***C07K 1/02*** *(2006.01)*

(86) International application number:
**PCT/EP2008/060494**

(87) International publication number:
**WO 2010/015287 (11.02.2010 Gazette 2010/06)**

## (54) Template-fixed peptidomimetics

Auf ein Gerüst Fixierte Peptidomimetika

Peptidomimétiques fixés sur matrice

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**25.05.2011 Bulletin 2011/21**

(73) Proprietors:
- **Polyphor AG**
  **4123 Allschwil (CH)**
- **Universität Zürich**
  **8006 Zürich (CH)**

(72) Inventors:
- **ROBINSON, John Anthony**
  **CH-8615 Wermatswil (CH)**
- **MÖHLE, Kerstin**
  **CH-8907 Wettswil (CH)**
- **SEITZ, Markus**
  **D-78464 (DE)**
- **MAILLARD, Ludovic T.**
  **F-34093 Montpellier (FR)**
- **MOUNME, Roba**
  **F-75015 Paris (FR)**
- **GOMBERT, Frank Otto**
  **CH-4102 Binningen (CH)**
- **SELLIER-KESSLER, Odile**
  **F-68390 Baldersheim (FR)**
- **HENZE, Heiko**
  **CH-8057 Zürich (CH)**
- **OBRECHT, Daniel**
  **CH-4112 Bättwil (CH)**
- **BISANG, Christian**
  **CH-4057 Basel (CH)**
- **LEDERER, Alexander**
  **CH-4053 Basel (CH)**

(74) Representative: **Braun, André jr. et al**
**Braunpat Braun Eder AG**
**Reussstrasse 22**
**4054 Basel (CH)**

(56) References cited:
**WO-A1-2004/096840 WO-A1-2006/087001**
**WO-A1-2007/079597 WO-A1-2008/092281**
**WO-A2-02/32932 WO-A2-03/031587**

- **RUDI FASAN, RICARDO L. A. DIAS ET AL.: "Structure-Activity Studies in a Family of beta-Hairpin Protein Epitope mimetic Inhibitors of the p53-HDM2 Protein-Protein Interaction" CHEMBIOCHEM, vol. 7, no. 3, 6 March 2006 (2006-03-06), pages 515-526, XP002569253**
- **LUJONG JIANG ET AL: "Combinatorial Biomimetic Chemistry: Parallel Synthesis of a Small Library of beta-Hairpin Mimmetics Based on Loop III from Human Platelet-Derived Growth Factor B" 1 January 2000 (2000-01-01), HELVETICA CHIMICA ACTA, VERLAG HELVETICA CHIMICA ACTA, BASEL, CH, PAGE (S) 3097 - 3112 , XP002202283 ISSN: 0018-019X Page 3110: Parallel Synthesis of Cyclo(-X1-X2-Val-Arg-Lys-Lys-X7-X8-D-Pro-P ro-)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- RUDI FASAN, RICARDO L. A. DIAS, KERSTIN MOEHLE, OLIVER ZERBE, JAN W. VRIJBLOED, DANIEL OBRECHT AND JOHN A. ROBINSON: "Using a beta-Hairpin To Mimic an alpha-Helix: Cyclic Peptidomimetic Inhibitors of the p53-HDM2 Protein-Protein Interaction" ANGEWANDTE CHEMIE INTERNATIONAL EDITION, vol. 43, no. 16, 13 April 2004 (2004-04-13), pages 2109-2112, XP002569254
- PETER M. FISCHER: "Peptide, Peptidomimetic, and Small-molecule Antagonists of the p53-HDM2 Protein-Protein Interaction" INTERNATIONAL JOURNAL OF PEPTIDE RESEARCH AND THERAPEUTICS, vol. 12, no. 1, March 2006 (2006-03), pages 3-19, XP002569255
- PFEIFER MARC E ET AL: "Synthesis and solution conformation of.beta.-hairpin mimetics utilizing a template derived from (2S,3R, 4R)-diaminoproline" HELVETICA CHIMICA ACTA, VERLAG HELVETICA CHIMICA ACTA, BASEL, CH, vol. 83, no. 2, 1 January 2000 (2000-01-01), pages 444-464, XP002180749 ISSN: 0018-019X
- MARKUS SEITZ, LUDOVIC T. MAILLARD, DANIEL OBRECHT, JOHN A. ROBINSON: "Molecular characterization of the NCoA-1-STAT6 Interaction" CHEMBIOCHEM, vol. 9, 23 May 2008 (2008-05-23), pages 1318-1322, XP008118881
- YUSUKE SAKO, ET AL: 'ribosomal Synthesis of Peptidase-Resistant Peptides Closed by a Nonreducible Inter-Side Chain Bond' ACS CHEMICAL BIOLOGY vol. 3, no. 4, 14 March 2008, pages 241 - 249

## Description

The present invention provides template-fixed beta-hairpin peptidomimetics incorporating a template-fixed chain of 8 alpha-amino acid residues which, depending on their positions in the chain, are Gly or Pro or of certain types, as defined herein below. These template-fixed
beta-hairpin mimetics have an agonizing or antagonizing activity against urotensin II, a G-protein-coupled receptor (GPCR). In addition, the present invention provides an efficient synthetic process by which these compounds can, if desired, be made in parallel library-format.

Many medically significant biological processes are mediated by signal transduction that involves GPCRs. The family of GPCRs includes receptors for hormones, neurotransmitters, growth factors and viruses (Th. Klabunde, G. Hessler, ChemBioChem 2002 3, 928-44). As for 210 receptors the natural ligand is known, another 150, so-called orphan receptors, have been identified within the human genome, for which the (patho)physiological function is unknown (A. Wise, S.C. Jupe, S. Rees, Annu. Rev. Pharmacol. Toxicol. 2004, 44, 43-66).

The GPCRs can be grouped into three major families: family A (rhodopsin-like or adrenergic-like family), family B (glucagon-receptor-like or secretin-receptor-like family) and family C (metabotropic glutamate receptors). Within each receptor family a certain sequence pattern (so-called fingerprint) and several structural features beyond the generally shared membrane topology are conserved (T.K. Attwood, Trends Pharmacol. Sci. 2001, 22, 165-65). Family A is by far the largest class. GPCRs are membrane-bound and characterized by a conserved seven helix transmembrane-spanning domain. As the first GPCR structure at atomic resolution, the 3D structure of bovine rhodopsin by X-ray crystallography was reported (K. Palczewsky et al. Science 2000, 289, 739-45). Based on this structure several models for other GPCRs have been reported using homology modeling (M.C. Gershengorn et al. Endocrinology 2001, 142, 2-10; S. Shacham et al. Med. Res. Rev. 2001, 21, 472-83). Recently, the crystal structure of the human $\beta_2$-adrenergic GPCR has been published (S.G. Rasmussen et al. Nature 2007, 450, 383-387).

Although over the past 15 years, nearly 350 therapeutic agents targeting GPCR receptors have been successfully introduced into the market (Th. Klabunde, G. Hessler, ChemBioChem 2002, 3, 928-44; G. Vauquelin et al. Fundam. Clin. Pharmacol. 2005, 19, 45-56), several toxicological problems which arose from mainly lack of selectivity of some of those drugs, need to be further investigated. WO0232932 discloses a cyclic urotensin II agonist. Clearly there is a need for new compounds for treating or preventing diseases including, but not limited to, infections, cancers, allergies, cardiovascular and peripheral and central nervous system disorder.

Transcription factors are central mediators of signal transduction. Manipulation of their activity by small molecules is a rapidly emerging area of both chemical biology and drug discovery (D. Ghosh, A.G. Papavassiliou, Curr. Med. Chem. 2005, 12, 691). One class of transcription factors contains signal transducer and activator of transcription (STAT) proteins, involved in many biological and medical relevant events, e.g. programmed cell death, organogenesis, innate and adaptive immunity or cell growth regulation (C.M. Horvath, TiBS, 2000, 25, 496). Transcription factors perform their function alone or by recruiting components of the transcription machinery to activate transcription. One type of these components are transcriptional coactivators.

Many drugs exert their effects through transcription factors whereof approximately 900 are associated with known diseases. Although transcription factors are key players in the pathogenesis of disease the complexity of the biology of transcriptional regulation still presents challenges to the discovery of new drugs as well as the design of therapies that directly target molecules involved in the transcription process. As the specificity of modulators plays a crucial role within therapeutic interventions as well there is clearly a need for new compounds for treating or preventing diseases including, but not limited to, various cancer like acute promyelocytic leukemia, breast cancer, endometrial cancer, prostate cancer, heptacellular carcinoma, metastasis, autoimmune diseases like airway hyperresponsiveness (AHR), eosinophilic inflammation, mucus production, asthma, neurodegenerative diseases, restinosis and gastrointestinal nematode parasites.

Herein described is a novel general approach to discover potent, selective and drugable ligands for GPCRs and modulators of transcriptional factors and coactivators. Within the scope of the present invention, this approach is particularly suited to discover ligands for peptidergic GPCRs as well as transcriptional coactivators.

Some of the peptidergic GPCR ligands/receptors interactions that are of therapeutic relevance are:

Somatostatins (A.V. Schally et al. Cell. Mol. Life Sci. 2004, 61, 1042-68), neurokinins, neurotensins (W. Rostène et al. Encyclop. Biol. Chem. 2004, 3, 3236; M. Boules et al. Expert. Opin. Investig. Drugs 2005, 14, 359-69; P. Kitabgi, Curr. Opin. Drug Disc. Devel. 2002, 5, 764-76), bradykinins (F. Marceau et al. Nat. Rev. Drug Disc. 2004, 3, 845-52), vasopressins (M. Ashton et al. Comb. Chem. And High Throughput Screening 2004, 7, 441-53), tachykinins, bombesins (E.R. Spindel et al. Recent Progress in Hormone Research 1993, 48, 365-91; R.T. Jensen et al. Growth Factors, Peptides, and Receptors, p. 225-237, Ed. By T. W. Moody, Plenum Press, New York, 1993; A.V. Schally et al. Cell. Mol. Life Sci. 2004, 61, 1042-68), endothelins (G. Ertl et al. Drugs 2004, 64, 1029-40), urotensin II (F.D. Russell, Pharmacol. Ther. 2004, 103, 223-43), GH-RH (A.V. Schally et al. Cell. Mol. Life Sci. 2004, 61, 1042-68), ghrelin

(A.V. Schally et al. Cell. Mol. Life Sci. 2004, 61, 1042-68; E. Ghio et al. Clin. Endocrinol. 2005, 62, 1-17), melanocortins (B.G. Irani et al. Curr. Pharm. Des. 2004, 10, 3443-79), glucagon-like peptide 1 (GLP-1, C.J. Small et al. Curr. Drug Targets CNS Neurol. Disord. 2004, 3, 379-88), peptide YY (PYY, C.J. Small et al. Curr. Drug Targets CNS Neurol. Disord. 2004, 3, 379-88), VIP (A.V. Schally et al. Cell. Mol. Life Sci. 2004, 61, 1042-68), and protease-activated receptors 1 and 2 (PAR-1 and 2, H.G. Selnick et al. Curr. Med. Chem. Cardiovasc. Hematol. Agents 2003, 1, 47-59; V.S. Ossovskaya et al. Physiol. Rev. 2004, 84, 579-621; A.M. Coelho et al. Curr. Med. Chem. Cardiovasc. Hematol. Agents 2003, 1, 61-72; M. Steinhoff et al. Endocrin. Rev. 2005, 26, 1-43).

Some of the transcription factor/transcriptional coactivator interactions that are of therapeutic relevance are:

HIF-1alpha/p300 (A.L. Kung, S.D. Zabludoff, D.S. France et al. Cancer Cell 2004, 6, 33), Tcf4/beta-catenin (M. Lepourcelet, Y.N.P. Chen, D.S. France et al. Cancer Cell 2004, 5, 91), ERalpha/SRC-2, ERbeta/SRC-2, TRbeta/SRC-2 (T.R. Geistlinger, R.K. Guy, J. Am. Chem. Soc. 2003, 125, 6852), ESX/Sur2 (H. Shimogawa, Y. Kwon, Q. Mao et al. J. Am. Chem. Soc. 2004, 126, 3461).

In the compounds described below, a new strategy is introduced to stabilize beta-hairpin conformations in backbone-turn peptidomimetics exhibiting selective agonizing or antagonizing activity against urotensin II. This involves transplanting the hairpin sequence onto a template, whose function is to restrain the peptide loop backbone into hairpin geometry.

Template-bound hairpin mimetic peptides have been described in the literature (D. Obrecht, M. Altorfer, J.A. Robinson, Adv. Med. Chem. 1999, 4, 1-68; J.A. Robinson, Syn. Lett. 2000, 4, 429-441), but such molecules have not previously been evaluated or disclosed for development of agonizing or antagonizing activity against urotensin II. However, the ability to generate beta-hairpin peptidomimetics using combinatorial and parallel synthesis methods has now been established (L. Jiang, K. Moehle, B. Dhanapal, D. Obrecht, J.A. Robinson, Helv. Chim. Acta 2000, 83, 3097-3112). These methods allow the synthesis and screening of large hairpin mimetic libraries, which in turn considerably facilitates structure-activity studies, and hence the discovery of new molecules with potent selective agonizing or antagonizing activity.

beta-Hairpin peptidomimetics obtained by the approach described here are useful for treating renal disease, diabetes, cardiovascular dysfunction, inflammation as well as allergic airways diseases like allergic rhinitis and asthma.

The beta-hairpin peptidomimetics of the present invention are compounds of the general formula

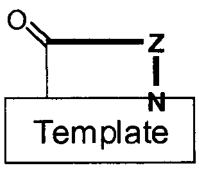

**(I)**

wherein

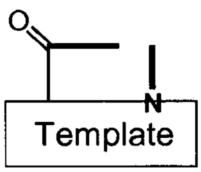

is $^{D}$Pro-$^{L}$Pro, $^{D}$Ser-LPro or $^{D}$Glu-$^{L}$Pro and

Z is a chain of 8 alpha-amino acid residues, the positions of said amino acid residues in said chain being counted starting from the N-terminal amino acid, whereby these amino acid residues are, depending on their position in the chain,

- P1: Asp;
- P2: Cys;
- P3: Phe, Tyr;
- P4: Trp, $^{D}$Trp;
- P5: Lys, Orn;

- P6: Tyr;
- P7: Cys,
- P8: Cha, Leu, Val; and
- Cys at P2 and P7 can form a disulfide bridge,

and pharmaceutically acceptable salts thereof.

In accordance with the present invention these beta-hairpin peptidomimetics can be prepared by a process which comprises

(a) coupling an appropriately functionalized solid support with an appropriately N-protected derivative of that amino acid which in the desired end-product is in positions 3, 4 or 5, any functional group which may be present in said N-protected amino acid derivative being likewise appropriately protected;
(b) removing the N-protecting group from the product thus obtained;
(c) coupling the product thus obtained with an appropriately N-protected derivative of that amino acid which in the desired end-product is one position nearer the N-terminal amino acid residue, any functional group which may be present in said N-protected amino acid derivative being likewise appropriately protected;
(d) removing the N-protecting group from the product thus obtained;
(e) repeating steps (c) and (d) until the N-terminal amino acid residue has been introduced;
(f) coupling the product thus obtained with a compound of the general formula

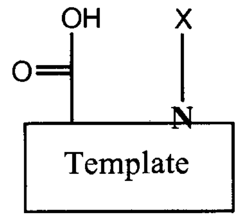

**II**

wherein

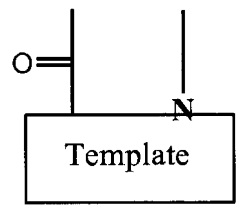

is as defined in claim 1 and X is an N-protecting group or, alternatively,

(fa) coupling the product obtained in step (e) with an appropriately N-protected derivative of an amino acid of the general formula

HOOC-B-H **III**

or

HOOC-A-H **IV**

wherein B and A are as defined in claim 1, any functional group which may be present in said N-protected amino acid derivative being likewise appropriately protected;
(fb) removing the N-protecting group from the product thus obtained; and
(fc) coupling the product thus obtained with an appropriately N-protected derivative of an amino acid of the above general formula IV or formula

HOOC-B3-H V

wherein B3 is as defined in claim 1

and, respectively, formula III, any functional group which may be present in said N-protected amino acid derivative being likewise appropriately protected;
(g) removing the N-protecting group from the product obtained in step (f) or (fc);
(h) coupling the product thus obtained with an appropriately N-protected derivative of that amino acid which in the desired end-product is in position, 8 any functional group which may be present in said N-protected amino acid derivative being likewise appropriately protected;
(i) removing the N-protecting group from the product thus obtained;
(j) coupling the product thus obtained with an appropriately N-protected derivative of that amino acid which in the desired end-product is one position farther away from position 8, any functional group which may be present in said N-protected amino acid derivative being likewise appropriately protected;
(k) removing the N-protecting group from the product thus obtained;
(l) repeating steps (j) and (k) until all amino acid residues have been introduced;
(m) if desired, selectively deprotecting one or several protected functional group(s) present in the molecule and appropriately substituting the reactive group(s) thus liberated;
(n) if desired, forming an interstrand linkage between side-chains of appropriate amino acid residues at positions 2 and 7;
(o) detaching the product thus obtained from the solid support;
(p) cyclizing the product cleaved from the solid support;
(q) removing any protecting groups present on functional groups of any members of the chain of amino acid residues and, if desired, any protecting group(s) which may in addition be present in the molecule; and
(r) if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

Alternatively, the peptidomimetics of the present invention can be prepared by

(a') coupling an appropriately functionalized solid support with a compound of the general formula

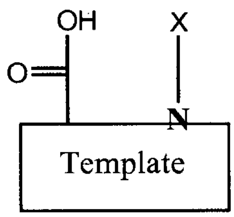

**II**

wherein

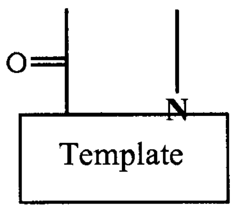

is as defined in claim 1 and X is an N-protecting group or, alternatively,

(a'a) coupling said appropriately functionalized solid support with an appropriately N-protected derivative of an amino acid of the general formula

HOOC-B-H **III**

or

HOOC-A-H **IV**

wherein B and A are as defined in claim 1, any functional group which may be present in said N-protected amino acid derivative being likewise appropriately protected;
(a'b) removing the N-protecting group from the product thus obtained; and
(a'c) coupling the product thus obtained with an appropriately N-protected derivative of an amino acid of the above general formula IV or formula

HOOC-B3-H **V**

wherein B3 is as defined in claim 1

and, respectively, formula III, any functional group which may be present in said N-protected amino acid derivative being likewise appropriately protected;
(b') removing the N-protecting group from the product obtained in step (a') or (a'c)
(c') coupling the product thus obtained with an appropriately N-protected derivative of that amino acid which in the desired end-product is in position 8, any functional group which may be present in said N-protected amino acid derivative being likewise appropriately protected;
(d') removing the N-protecting group from the product thus obtained;
(e') coupling the product thus obtained with an appropriately N-protected derivative of that amino acid which in the desired end-product is one position farther away from position 8, any functional group which may be present in said N-protected amino acid derivative being likewise appropriately protected;
(f) removing the N-protecting group from the product thus obtained;
(g') repeating steps (e') and (f) until all amino acid residues have been introduced;
(h') if desired, selectively deprotecting one or several protected functional group(s) present in the molecule and appropriately substituting the reactive group(s) thus liberated;
(i') if desired forming an interstrand linkage between side-chains of appropriate amino acid residues at opposite positions 2 and 7;
(j') detaching the product thus obtained from the solid support;
(k') cyclizing the product cleaved from the solid support;
(l') removing any protecting groups present on functional groups of any members of the chain of amino acid residues and, if desired, any protecting group(s) which may in addition be present in the molecule; and
(m') if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

The peptidomimetics of the present invention can also be enantiomers of the compounds of formula I. These enantiomers can be prepared by a modification of the above processes in which enantiomers of all chiral starting materials are used.

As used in this description, the term "alkyl", taken alone or in combinations, designates saturated, straight-chain or branched hydrocarbon radicals having up to 24, preferably up to 12, carbon atoms. Similarly, the term "alkenyl" designates straight chain or branched hydrocarbon radicals having up to 24, preferably up to 12, carbon atoms and containing at least one or, depending on the chain length, up to four olefmic double bonds. The term "lower" designates radicals and compounds having up to 6 carbon atoms. Thus, for example, the terms "lower alkyl" and "lower cycloalkyl" designate saturated, straight-chain or branched and, respectively cyclic hydrocarbon radicals having up to 6 carbon atoms, such as methyl, ethyl, n-propyl, isopropyl, n-butyl, sec.-butyl, isobutyl, tert.-butyl, cyclopentyl, cyclohexyl and the like. The term "aryl" designates aromatic carbocyclic hydrocarbon radicals containing one or two six-membered rings, such as phenyl or naphthyl, which may be substituted by up to three substituents such as Br, Cl, F, $CF_3$, $NO_2$, lower alkyl or lower alkenyl. The term "heteroaryl" designates aromatic heterocyclic radicals containing one or two five- and/or six-membered rings, at least one of them containing up to three heteroatoms selected from the group consisting of O, S and N and said ring(s) being optionally substituted; representative examples of such optionally substituted heteroaryl radicals are indicated hereinabove in connection with the definition of R".

The beta-hairpin conformation is highly relevant for the agonizing or antagonizing activity against urotensin II of the beta-hairpin mimetics of the present invention. The beta-hairpin stabilizing conformational properties of the templates play a key role not only for the selective activities described above but also for the synthesis process defined hereinabove, as incorporation of the templates at the beginning or near the middle of the linear protected peptide

precursors enhances cyclization yields significantly.

As mentioned earlier, positions for interstrand linkages are positions P2 and P7; taken together. Such interstrand linkages are known to stabilize the beta-hairpin conformations and thus constitute an important structural element for the design of beta-hairpin mimetics.

Most preferred amino acid residues in chain Z are those derived from natural alpha-amino acids. Hereinafter follows a list of amino acids which, or the residues of which, are suitable for the purposes of the present invention, the abbreviations corresponding to generally adopted usual practice:

| three letter code | | one letter code |
|---|---|---|
| Ala | L-Alanine | A |
| $^{D}$Ala | D-Alanine | $^{D}$A |
| Arg | L-Arginine | R |
| Asn | L-Asparagine | N |
| Asp | L-Aspartic acid | D |
| Cha | L-3-Cyclohexylalanine | |
| Cys | L-Cysteine | C |
| Glu | L-Glutamic acid | E |
| Glu(cHx) | L-Glutamic acid cyclohexyl ester | |
| Gln | L-Glutamine | Q |
| Gly | Glycine | G |
| His | L-Histidine | H |
| Ile | L-Isoleucine | I |
| Leu | L-Leucine | L |
| Lys | L-Lysine | K |
| Met | L-Methionine | M |
| Orn | L-Ornithine | |
| Phe | L-Phenylalanine | F |
| Pro | L-Proline | P |
| $^{D}$Pro | D-Proline | $^{D}$P |
| Ser | L-Serine | S |
| Thr | L-Threonine | T |
| Trp | L-Tryptophan | W |
| $^{D}$Trp | D-Tryptophan | $^{D}$W |
| Trp(6Cl) | 6-Chloro-L-Tryptophan | |
| Tyr | L-Tyrosine | Y |
| Val | L-Valine | V |

The peptidic chain **Z** within the beta-hairpin mimetics of the invention comprises 8 amino acid residues. The positions P1 to P8 of each amino acid residue in the chain **Z** are unequivocally defined as follows: P1 represents the first amino acid in the chain **Z** that is coupled with its N-terminus to the C-terminus of the templates $^{D}$Pro-$^{L}$Pro, $^{D}$Ser-$^{L}$Pro or $^{D}$Glu-$^{L}$Pro; and P8 represents the last amino acid in the chain Z that is coupled with its C-terminus to the N-terminus of the said templates

For the β-peptidomimetics having an agonizing or antagonizing activity against urotensin II the alpha-amino acid residues in positions 1 to 8 of the chain Z are:

- P1: Asp;
- P2: Cys;
- P3: Phe, Tyr;
- P4: Trp, $^{D}$Trp;
- P5: Lys, Orn;
- P6: Tyr;
- P7: Cys,
- P8: Cha, Leu, Val; and
  Cys at P2 and P7 may form a disulfide bridge.

Particularly preferred beta-peptidomimetics of the invention include those described in Examples 31 and 32.

The processes of the invention can advantageously be carried out as parallel array syntheses to yield libraries of template-fixed beta-hairpin peptidomimetics of the above general formula **I.** Such parallel syntheses allow one to obtain arrays of numerous (normally 12 to 192, typically 96) compounds of general formula I in high yields and defined purities, minimizing the formation of dimeric and polymeric by-products. The proper choice of the functionalized solid-support (i.e. solid support plus linker molecule), templates and site of cyclization play thereby key roles.

The functionalized solid support is conveniently derived from polystyrene crosslinked with, preferably 1-5%, divinylbenzene; polystyrene coated with polyethyleneglycol spacers (Tentagel$^R$); and polyacrylamide resins (see also D. Obrecht, J.-M. Villalgordo, "Solid- Supported Combinatorial and Parallel Synthesis of Small-Molecular-Weight Compound Libraries", Tetrahedron Organic Chemistry Series, Vol. 17, Pergamon, Elsevier Science, 1998**).**

The solid support is functionalized by means of a linker, i.e. a bifunctional spacer molecule which contains on one end an anchoring group for attachment to the solid support and on the other end a selectively cleavable functional group used for the subsequent chemical transformations and cleavage procedures. For the purposes of the present invention two types of linkers can be used:

Type 1 linkers are designed to release the amide group under acid conditions (H. Rink, Tetrahedron Lett. 1987, 28, 3783-3790). Linkers of this kind form amides of the carboxyl group of the amino acids; examples of resins functionalized by such linker structures include 4-[(((2,4-dimethoxyphenyl)Fmoc-aminomethyl)phenoxyacetamido) aminomethyl] PS resin, 4-[(((2,4-dimethoxyphenyl)Fmoc-aminomethyl)phenoxyacetamido) aminomethyl]-4-methylbenzydrylamine PS resin (Rink amide MBHA PS Resin), and 4-[(((2,4-dimethoxyphenyl)Fmoc-aminomethyl)phenoxyacetamido) aminomethyl] benzhydrylamine PS-resin (Rink amide BHA PS resin). Preferably, the support is derived from polystyrene crosslinked with, most preferably 1-5%, divinylbenzene and functionalized by means of the 4-(((2,4-dimethoxyphenyl)Fmoc-aminomethyl)phenoxyacetamido) linker.

Type 2 linkers are designed to eventually release the carboxyl group under acidic conditions. Linkers of this kind form acid-labile esters with the carboxyl group of the amino acids, usually acid-labile benzyl, benzhydryl and trityl esters; examples of such linker structures include 2-methoxy-4-hydroxymethylphenoxy (Sasrin$^R$ linker), 4-(2,4-dimethoxyphenyl-hydroxymethyl)-phenoxy (Rink linker), 4-(4-hydroxymethyl-3-methoxyphenoxy)butyric acid (HMPB linker), trityl and 2-chlorotrityl. Preferably, the support is derived from polystyrene crosslinked with, most preferably 1-5%, divinylbenzene and functionalized by means of the 2-chlorotrityl linker.

When carried out as parallel array syntheses the processes of the invention can be advantageously carried out as described herein below but it will be immediately apparent to those skilled in the art how these procedures will have to be modified in case it is desired to synthesize one single compound of the above formula I.

A number of reaction vessels (normally 12 to 192, typically 96) equal to the total number of compounds to be synthesized by the parallel method are loaded with 25 to 1000 mg, preferably 60 mg, of the appropriate functionalized solid support, preferably 1 to 3% cross-linked polystyrene or Tentagel resin.

The solvent to be used must be capable of swelling the resin and includes, but is not limited to, dichloromethane (DCM), dimethylformamide (DMF), N-methylpyrrolidone (NMP), dioxane, toluene, tetrahydrofuran (THF), ethanol (EtOH), trifluoroethanol (TFE), isopropylalcohol and the like. Solvent mixtures containing as at least one component a polar solvent (e. g. 20% TFE/DCM, 35% THF/NMP) are beneficial for ensuring high reactivity and solvation of the resin-bound peptide chains (G.B. Fields, C.G. Fields, J. Am. Chem. Soc. 1991, 113, 4202-4207).

With the development of various linkers that release the C-terminal carboxylic acid group under mild acidic conditions, not affecting acid-labile groups protecting functional groups in the side chain(s), considerable progresses have been made in the synthesis of protected peptide fragments. The 2-methoxy-4-hydroxybenzylalcohol-derived linker (SasrinR linker, Mergler et al. Tetrahedron Lett. 1988, 29 4005-4008) is cleavable with diluted trifluoroacetic acid (0.5-1% TFA in DCM) and is stable to Fmoc deprotection conditions during the peptide synthesis, Boc/tBu-based additional protecting groups being compatible with this protection scheme. Other linkers which are suitable for the process of the invention include the super acid labile 4-(2,4-dimethoxyphenyl-hydroxymethyl)-phenoxy linker (Rink linker, H. Rink, Tetrahedron Lett. 1987, 28, 3787-3790), where the removal of the peptide requires 10% acetic acid in DCM or 0.2% trifluoroacetic acid in DCM; the 4-(4-hydroxymethyl-3-methoxyphenoxy)butyric acid-derived linker (HMPB-linker, Flörsheimer & Riniker, Peptides 1991, 1990 131) which is also cleaved with 1%TFA/DCM in order to yield a peptide fragment containing all acid labile side- chain protective groups; and, in particular, the 2-chlorotritylchloride linker (Barlos et al. Tetrahedron Lett. 1989, 30, 3943-3946), which allows the peptide detachment using a mixture of glacial acetic acid/trifluoroethanol/DCM (1:2:7) for 30 min.

Suitable protecting groups for amino acids and, respectively, for their residues are, for example,

- for the amino group (as is present e. g. also in the side-chain of lysine)

| | |
|---|---|
| Cbz | benzyloxycarbonyl |
| Boc | tert.-butyloxycarbonyl |
| Fmoc | 9-fluorenylmethoxycarbonyl |
| Alloc | allyloxycarbonyl |
| Teoc | trimethylsilylethoxycarbonyl |
| Tcc | trichloroethoxycarbonyl |
| Nps | o-nitrophenylsulfonyl; |
| Trt | triphenymethyl or trityl; |

- for the carboxyl group (as is present e. g. also in the side-chain of aspartic and glutamic acid) by conversion into esters with the alcohol components

| | |
|---|---|
| tBu | tert.-butyl |
| Bn | benzyl |
| Me | methyl |
| Ph | phenyl |
| Pac | Phenacyl |
| | Allyl |
| Tse | trimethylsilylethyl |
| Tce | trichloroethyl; |

- for the guanidino group (as is present e. g. in the side-chain of arginine)

| | |
|---|---|
| Pmc | 2,2,5,7,8-pentamethylchroman-6-sulfonyl; |
| Ts | tosyl (i. e. p-toluenesulfonyl); |
| Cbz | benzyloxycarbonyl; |
| Pbf | pentamethyldihydrobenzofuran-5-sulfonyl; |

- for the hydroxy group (as is present e. g. in the side-chain of threonine and serine)

| | |
|---|---|
| tBu | tert.-butyl; |
| Bn | benzyl; |
| Trt | trityl; |

- and for the mercapto group (as is present e. g. in the side-chain of cysteine)

| | |
|---|---|
| Acm | acetamidomethyl; |
| tBu | tert.-butyl; |
| Bn | benzyl; |
| Trt | trityl; and |
| Mtr | 4-methoxytrityl. |

The 9-fluorenylmethoxycarbonyl- (Fmoc)-protected amino acid derivatives are preferably used as the building blocks for the construction of the template-fixed beta-hairpin loop mimetics of formula I. For the deprotection, i. e. cleaving off of the Fmoc group, 20% piperidine in DMF or 2% DBU (1,8-Diazabicyclo[5.4.0]undec-7-ene)/2% piperidine in DMF can be used.

The quantity of the reactant, i. e. of the amino acid derivative, is usually 1 to 20 equivalents based on the milliequivalents per gram (meq/g) loading of the functionalized solid support (typically 0.1 to 2.85 meq/g for polystyrene resins) originally weighed into the reaction tube. Additional equivalents of reactants can be used, if required, to drive the reaction to completion in a reasonable time. The preferred workstations (without, however, being limited thereto) are Labsource's Combi-chem station, Protein Technologies' Symphony and MultiSyn Tech's-Syro synthesizer, the latter additionally equipped with a transfer unit and a reservoir box during the process of detachment of the fully protected linear peptide from the solid support. All synthesizers are able to provide a controlled environment; for example, reactions can be accomplished at temperatures different from room temperature as well as under inert gas atmosphere, if desired.

Amide bond formation requires the activation of the alpha-carboxyl group for the acylation step. When this activation is being carried out by means of the commonly used carbodiimides such as dicyclohexylcarbodiimide (DCC, Sheehan & Hess, J. Am. Chem. Soc. 1955, 77, 1067-1068) or diisopropylcarbodiimide (DIC, Sarantakis et al Biochem. Biophys. Res. Commun. 1976, 73, 336-342), the resulting dicyclohexylurea and, respectively, diisopropylurea is insoluble

and, respectively, soluble in the solvents generally used. In a variation of the carbodiimide method 1-hydroxybenzotriazole (HOBt, König & Geiger, Chem. Ber 1970, 103, 788-798) is included as an additive to the coupling mixture. HOBt prevents dehydration, suppresses racemization of the activated amino acids and acts as a catalyst to improve the sluggish coupling reactions. Certain phosphonium reagents have been used as direct coupling reagents, such as benzotriazol-1-yl-oxy-tris-(dimethylamino)-phosphonium hexafluorophosphate (BOP, Castro et al. Tetrahedron Lett. 1975, 14, 1219-1222; Synthesis 1976, 751-752), or benzotriazol-1-yl-oxy-tris-pyrrolidino-phosphonium hexaflurophoshate (Py-BOP, Coste et al. Tetrahedron Lett. 1990, 31, 205-208), or 2-(1H-benzotriazol-1-yl-)1,1,3,3-tetramethyluronium tetrafluoroborate (TBTU), or hexafluorophosphate (HBTU, Knorr et al. Tetrahedron Lett. 1989, 30, 1927-1930); these phosphonium reagents are also suitable for in situ formation of HOBt esters with the protected amino acid derivatives. More recently diphenoxyphosphoryl azide (DPPA) or O-(7-aza-benzotriazol-1-yl)-N,N,N',N'-tetramethyluronium tetrafluoroborate (TATU) or O-(7-aza-benzotriazol-1-yl)-N,N,N',N'-tetramethyluronium hexafluorophosphate (HATU)/7-aza-1-hydroxy benzotriazole (HOAt, Carpino et al. Tetrahedron Lett. 1994, 35, 2279-2281) or -(6-chloro-1H-benzotriazol-1-yl-)-N,N,N',N'-1,1,3,3-tetramethyluronium tetrafluoroborate (TCTU), or hexafluorophosphate (HCTU, Marder, Shivo and Albericio: HCTU and TCTU: New Coupling Reagents: Development and Industrial Applications, Poster Presentation, Gordon Conference February 2002) have also been used as coupling reagents.

Due to the fact that near-quantitative coupling reactions are essential, it is desirable to have experimental evidence for completion of the reactions. The ninhydrin test (Kaiser et al. Anal. Biochemistry 1970, 34, 595*),* where a positive colorimetric response to an aliquot of resin-bound peptide indicates qualitatively the presence of the primary amine, can easily and quickly be performed after each coupling step. Fmoc chemistry allows the spectrophotometric detection of the Fmoc chromophore when it is released with the base (Meienhofer et al. Int. J. Peptide Protein Res. 1979, 13, 35-42).

The resin-bound intermediate within each reaction vessel is washed free of excess of retained reagents, of solvents, and of by-products by repetitive exposure to pure solvent(s) by one of the two following methods:

1) The reaction vessels are filled with solvent (preferably 5 ml), agitated for 5 to 300 minutes, preferably 15 minutes, and drained to expel the solvent;

2) The reaction vessels are filled with solvent (preferably 5 ml) and drained into a receiving vessel such as a test tube or vial.

Both of the above washing procedures are repeated up to about 50 times (preferably about 10 times), monitoring the efficiency of reagent, solvent, and by-product removal by methods such as TLC, GC, or inspection of the washings.

The above described procedure of reacting the resin-bound compound with reagents within the reaction tubes followed by removal of excess reagents, by-products, and solvents is repeated with each successive transformation until the final resin-bound fully protected linear peptide has been obtained.

Before this fully protected linear peptide is detached from the solid support, it is possible, if desired, to selectively deprotect one or several protected functional group(s) present in the molecule and to appropriately substitute the reactive group(s) thus liberated. To this effect, the functional group(s) in question must initially be protected by a protecting group which can be selectively removed without affecting the remaining protecting groups present. Alloc (allyloxycarbonyl) is an example for such an amino protecting group which can be selectively removed, e.g. by means of $Pd^0$ and phenylsilane in $CH_2Cl_2$, without affecting the remaining protecting groups, such as Fmoc, present in the molecule. The reactive group thus liberated can then be treated with an agent suitable for introducing the desired substituent. Thus, for example, an amino group can be acylated by means of an acylating agent corresponding to the acyl substituent to be introduced.

Before this fully protected linear peptide is detached from the solid support, it is also possible, if desired, to form an interstrand linkage between side-chains of appropriate amino acid residues at positions 2 and 7.

Interstrand linkages and their formation have been discussed above, in connection with the explanations made regarding groups of the type H which can, for example, be disulfide bridges formed by cysteine and homocysteine residues at positions 2 and 7; or lactam bridges formed by glutamic and aspartic acid residues linking ornithine and, respectively, lysine residues, or by glutamic acid residues linking 2,4-diaminobutyric acid residues located at positions 2 and 7 by amide bond formation. The formation of such interstrand linkages can be effected by methods well known in the art.

For the formation of disulfide bridges preferably a solution of 10 equivalents of iodine solution is applied in DMF or in a mixture of $CH_2Cl_2$ /MeOH for 1.5 h which is repeated for another 3h with a fresh iodine solution after filtering of the iodine solution, or in a mixture of DMSO and acetic acid solution, buffered with 5% with $NaHCO_3$ to pH 5-6 for 4h, or in water after adjusted to pH 8 with ammonium hydroxide solution by stirring for 24h, or in a solution ofNMP and tri-n- butylphosphine (preferably 50 eq.).

For the formation of lactam bridges preferably a solution of 2 equivalents of HATU (*N*-[(dimethylamino)-1*H*-1,2,3-triazolo[4,5-*b*]pyridin-1-ylmethylene]-*N*-methyl-methanaminium hexafluorophosphate *N*-oxide) in dry DMF and a

solution of 4 equivalents of DIPEA (Diisopropyl ethaylamine) in dry DMF is applied for 16 h.

Detachment of the fully protected linear peptide from the solid support is achieved by exposing the loaded resin with a solution of the cleavage reagent (preferably 3 to 5 ml). Temperature control, agitation, and reaction monitoring are implemented as described above. Via a transfer-unit the reaction vessels are connected with a reservoir box containing reservoir tubes to efficiently collect the cleaved product solutions. The resins remaining in the reaction vessels are then washed 2 to 5 times as above with 3 to 5 ml of an appropriate solvent to extract (wash out) as much of the detached products as possible. The product solutions thus obtained are combined, taking care to avoid cross-mixing. The individual solutions/extracts are then manipulated as needed to isolate the final compounds. Typical manipulations include, but are not limited to, evaporation, concentration, liquid/liquid extraction, acidification, basification, neutralization or additional reactions in solution.

The solutions containing fully protected linear peptide derivatives which have been cleaved off from the solid support and neutralized with a base, are evaporated. Cyclization is then effected in solution using solvents such as DCM, DMF, dioxane, THF and the like. Various coupling reagents which were mentioned earlier can be used for the cyclization. The duration of the cyclization is about 6-48 hours, preferably about 16 hours. The progress of the reaction is followed, e. g. by RP-HPLC (Reverse Phase High Performance Liquid Chromatography). Then the solvent is removed by evaporation, the fully protected cyclic peptide derivative is dissolved in a solvent which is not miscible with water, such as DCM, and the solution is extracted with water or a mixture of water-miscible solvents, in order to remove any excess of the coupling reagent.

Alternatively, the detachment and complete deprotection of the fully protected peptide from the solid support can be achieved manually in glass vessels.

Finally, the fully protected peptide derivative is treated with 95% TFA, 2.5% $H_2O$, 2.5% TIS or another combination of scavengers for effecting the cleavage of protecting groups. The cleavage reaction time is commonly 30 minutes to 12 hours, preferably about 2.5 hours.

After fully deprotection one of the following methods can be used for further work-up:

1) The volatiles are evaporated to dryness and the crude peptide is dissolved in 20% AcOH in water and extracted with isopropyl ether or other solvents which are suitable therefore. The aqueous layer is collected and evaporated to dryness, and the fully deprotected cyclic peptide derivative of formula I is obtained as end-product;

2) The fully deprotection mixture is concentrated under vacuum. Following precipitation of the fully deprotected peptide in diethylether at preferably 0°C the solid is washed up to about 10 times, preferably 3 times, dried, and the fully deprotected cyclic peptide derivative of formula I is obtained as end-product.

As mentioned earlier, it is thereafter possible, if desired, to convert a fully deprotected product of formula I thus obtained into a pharmaceutically acceptable salt or to convert a pharmaceutically acceptable, or unacceptable, salt thus obtained into the corresponding free compound of formula I or into a different, pharmaceutically acceptable, salt. Any of these operations can be carried out by methods well known in the art.

The template starting materials of formula II used in the processes of the invention, pre-starting materials therefore, and the preparation of these starting and pre-starting materials are described in International Application PCT/EP02/01711 of the same applicants, published as WO 02/070547 A1.

The beta-hairpin peptidomimetics of the invention can be used in a wide range of applications in order to treat, in particular (but not limited thereto), renal diseases, cardiorenal diseases, diabetes, inflammation, heart failure, hypertension, endothelial dysfunction, insulin resistance, hyperglycemia, allergic reactions including asthma and atopic diseases,.

These beta-hairpin peptidomimetics may be administered per se or may be applied as an appropriate formulation together with carriers, diluents or excipients well known in the art.

They can be administered singly, as mixtures of several of these beta-hairpin peptidomimetics or in combination with other pharmaceutically active agents such as antiinflammatory agents or antimicrobial agents or anti cancer agents or anti- HIV agents.

Pharmaceutical compositions comprising beta-hairpin peptidomimetics of the invention may be manufactured by means of conventional mixing, dissolving, granulating, coated tablet-making, levigating, emulsifying, encapsulating, entrapping or lyophilizing processes. Pharmaceutical compositions may be formulated in conventional manner using one or more physiologically acceptable carriers, diluents, excipients or auxiliaries which facilitate processing of the active beta-hairpin peptidomimetics into preparations which can be used pharmaceutically. Proper formulation depends upon the method of administration chosen.

For topical administration the beta-hairpin peptidomimetics of the invention may be formulated as solutions, gels, ointments, creams, suspensions, etc. as are well-known in the art.

Systemic formulations include those designed for administration by injection, e.g. subcutaneous, intravenous, intramuscular, intrathecal or intraperitoneal injection, as well as those designed for transdermal, transmucosal, oral or

pulmonary administration.

For injections, the beta-hairpin peptidomimetics of the invention may be formulated in adequate solutions, preferably in physiologically compatible buffers such as Hink's solution, Ringer's solution, or physiological saline buffer. The solutions may contain formulatory agents such as suspending, stabilizing and/or dispersing agents. Alternatively, the beta-hairpin peptidomimetics of the invention may be in powder form for combination with a suitable vehicle, e.g., sterile pyrogen-free water, before use.

For transmucosal administration, penetrants appropriate to the barrier to be permeated are used in the formulation as known in the art.

For oral administration, the compounds can be readily formulated by combining the active beta-hairpin peptidomimetics of the invention with pharmaceutically acceptable carriers well known in the art. Such carriers enable the beta-hairpin peptidomimetics of the invention to be formulated as tablets, pills, dragees, capsules, liquids, gels, syrups, slurries, suspensions etc., for oral ingestion by a patient to be treated. For oral formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl cellulose, sodium carboxymethylcellulose, and/or polyvinylpyrrolidone (PVP); granulating agents; and binding agents. If desired, desintegrating agents may be added, such as cross-linked polyvinylpyrrolidones, agar, or alginic acid or a salt thereof, such as sodium alginate. If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, etc. In addition, flavoring agents, preservatives, coloring agents and the like may be added.

For buccal administration, the composition may take the form of tablets, lozenges, etc. formulated as usual.

For administration by inhalation, the beta-hairpin peptidomimetics of the invention are conveniently delivered in form of an aeorosol spray from pressurized packs or a nebulizer, with the use of a suitable propellant, e.g. dichlorodifluoromethane, trichlorofluromethane, carbon dioxide or another suitable gas. In the case of a pressurized aerosol the dose unit may be determined by providing a valve to deliver a metered amount. Capsules and cartridges of e.g. gelatin for use in an inhaler or insufflator may be formulated containing a powder mix of the beta-hairpin peptidomimetics of the invention and a suitable powder base such as lactose or starch.

The compounds may also be formulated in rectal or vaginal compositions such as suppositories together with appropriate suppository bases such as cocoa butter or other glycerides.

In addition to the formulations described above, the beta-hairpin peptidomimetics of the invention may also be formulated as depot preparations. Such long acting formulations may be administered by implantation (e.g. subcutaneously or intramuscularly) or by intramuscular injection. For the manufacture of such depot preparations the beta-hairpin peptidomimetics of the invention may be formulated with suitable polymeric or hydrophobic materials (e.g. as an emulsion in an acceptable oil) or ion exchange resins, or as sparingly soluble salts.

In addition, other pharmaceutical delivery systems may be employed such as liposomes and emulsions as well known in the art. Certain organic solvents such as dimethylsulfoxide may also be employed. Additionally, the beta-hairpin peptidomimetics of the invention may be delivered using a sustained-release system, such as semipermeable matrices of solid polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic agent, additional strategies for protein stabilization may be employed.

As the beta-hairpin pepdidomimetics of the invention may contain charged residues, they may be included in any of the above-described formulations as such or as pharmaceutically acceptable salts. Pharmaceutically acceptable salts tend to be more soluble in aqueous and other protic solvents than are the corresponding free forms.

The beta-hairpin peptidomimetics of the invention, or compositions thereof, will generally be used in an amount effective to achieve the intended purpose. It is to be understood that the amount used will depend on a particular application.

For topical administration a therapeutically effective dose can be determined using, for example, the in vitro assays provided in the examples. An ordinary skilled expert will be able to determine therapeutically effective amounts without undue experimentation.

For systemic administration, a therapeutically effective dose can be estimated initially from in vitro assays. For example, a dose can be formulated in animal models to achieve a circulating beta-hairpin peptidomimetic concentration range that includes the $IC_{50}$ as determined in the cell culture (i.e. the concentration of a test compound that is lethal to 50% of a cell culture). Such information can be used to more accurately determine useful doses in humans.

Initial dosages can also be determined from in vivo data, e.g. animal models, using techniques that are well known in the art. One having ordinary skill in the art could readily optimize administration to humans based on animal data.

Dosage amounts may be adjusted individually to provide plasma levels of the beta-hairpin peptidomimetics of

the invention which are sufficient to maintain the therapeutic effect. Therapeutically effective serum levels may be achieved by administering multiple doses each day.

In cases of local administration or selective uptake, the effective local concentration of the beta-hairpin peptidomimetics of the invention may not be related to plasma concentration. One having the ordinary skill in the art will be able to optimize therapeutically effective local dosages without undue experimentation.

The amount of beta-hairpin peptidomimetics administered will, of course, be dependent on the subject being treated, on the subject's weight, the severity of the affliction, the manner of administration and the judgement of the prescribing physician.

Normally, a therapeutically effective dose of the beta-hairpin peptidomimetics described herein will provide therapeutic benefit without causing substantial toxicity.

Toxicity of the beta-hairpin peptidomimetics of the invention can be determined by standard pharmaceutical procedures in cell cultures or experimental animals, e.g., by determining the $LD_{50}$ (the dose lethal to 50% of the population) or the $LD_{100}$ (the dose lethal to 100% of the population). The dose ratio between toxic and therapeutic effect is the therapeutic index. Compounds which exhibit high therapeutic indices are preferred. The data obtained from these cell culture assays and animal studies can be used in formulating a dosage range that is not toxic for use in humans. The dosage of the beta-hairpin peptidomimetics of the invention lies preferably within a range of circulating concentrations that include the effective dose with little or no toxicity. The dosage may vary within the range depending upon the dosage form employed and the route of administration utilized. The exact formulation, route of administration and dose can be chosen by the individual physician in view of the patient's condition (see, e.g. Fingl et al. 1975, in: The Pharmacological Basis of Therapeutics, Ch.1, p.1).

The following Examples illustrate the present invention.

## Examples

### 1. Peptide Synthesis

*Coupling of the first protected amino acid residue to the resin*

1 g (1.4 mMol) of 2-chlorotritylchloride resin (1.4 mMol/g; Barlos et al. Tetrahedron Lett. 1989, 30, 3943-3946) was filled into a dried flask. The resin was suspended in $CH_2Cl_2$ (5 ml) and allowed to swell at room temperature under constant shaking for 30 min. A solution of 0.98mMol (0.7 eq) of the first suitably protected amino acid residue (see below) in $CH_2Cl_2$ (5 ml) completed by 960 μl (4 eq) of diisopropylethylamine (DIEA) was added. After shaking the reaction mixture for 4 hours at 25 °C the resin was filtered and washed successively with $CH_2Cl_2$ (1x), DMF (1x) and $CH_2Cl_2$ (1x). A solution of $CH_2Cl_2$/MeOH/DIEA (17/2/1, 10 ml) was added to the resin and the suspension was shaken for 30 min. After filtration the resin was washed in the following order with $CH_2Cl_2$ (1x), DMF (1x), $CH_2Cl_2$ (1x), MeOH (1x), $CH_2Cl_2$ (1x), MeOH (1x), $CH_2Cl_2$ (2x), $Et_2O$ (2x) and dried under vacuum for 6 hours.
Loading was typically 0.6-0.7 mMol/g.

The following preloaded resins were prepared: Fmoc-ProO-chlorotritylresin, Fmoc-4Hyp2(tBu)O-chlorotritylresin, Fmoc-OicO-chlorotritylresin, and Fmoc-4Mp1(Trt)O-chloro-tritylresin.

The synthesis was carried out employing a Syro-peptide synthesizer (MultiSynTech) using 24-96 reaction vessel. In each vessel 0.04 mMol of the above resin were placed and the resin was swollen in $CH_2Cl_2$ and DMF for 15 min, respectively. The following reaction cycles were programmed and carried out:

| Step | Reagent | Time |
|---|---|---|
| 1 | DMF, wash and swell | 2 x 1 min |
| 2 | 20% piperidine/DMF | 1 x 5 min, 1 x 15 min |
| 3 | DMF, wash | 5 x 1 min |
| 4a | 5 eq Fmoc amino acid/DMF + 5 eq HCTU/DMF, 10 eq DIEA | 1 x 60 min |
| 5 | DMF, wash | 3 x 1 min |

Step 4a was repeated once.

If not indicated otherwise, the final coupling of an amino acid is followed by an Fmoc deprotection by applying steps 1-3 of the above described reaction cycle.

The following Fmoc-protected amino acid derivative had to be synthesized before its usage in the linear peptide synthesis described above.

*Synthesis of N-Fmoc-protected L-6-chlorotryptophan*

(modified procedure following E. Atherton, R. Sheppard, Solid phase peptide synthesis. A practical approach, IRL Press, Oxford, 1989, page 49).

**Cyclization and work up of backbone cyclized peptides**

*Cleavage of the fully protected peptide fragment*

After completion of the synthesis, the resin (0.04 mMol) was suspended in 1 ml (0.13 mMol, 3.4 eq) of 1% TFA in $CH_2Cl_2$ (*v*/*v*) for 3 minutes, filtered, and the filtrate was neutralized with 1 ml (0.58 mMol, 14.5 eq) of 10% DIEA in $CH_2Cl_2$ (v/v). This procedure was repeated three times to ensure completion of the cleavage. The filtrate was evaporated to dryness and a sample of the product was fully deprotected by using a cleavage mixture containing 95 % trifluoroacetic acid (TFA), 2.5 % water and 2.5 % triisopropylsilane (TIS) to be analyzed by reverse phase-HPLC (column $C_{18}$) and ESI-MS to monitor the efficiency of the linear peptide synthesis.

*Cyclization of the linear peptide*

The fully protected linear peptide (0.04 mMol) was dissolved in DMF (4 μMol/ml). Then 30.4 mg (0.08 mMol, 2 eq) of HATU, 10.9 mg (0.08 mMol, 2 eq) of HOAt and 28 μl (0.16 mMol, 4 eq) DIEA were added, and the mixture was vortexed at 25°C for 16 hours and subsequently concentrated under high vacuum. The residue was partitioned between $CH_2Cl_2$ and $H_2O/CH_3CN$ (90/10; *v*/*v*). The $CH_2Cl_2$ phase was evaporated to yield the fully protected cyclic peptide.

*Fully deprotecting the cyclic peptide*

The cyclic peptide obtained was dissolved in 3 ml of the cleavage mixture containing 82.5 % trifluoroacetic acid (TFA), 5 % water, 5 % thioanisole, 5 % phenol and 2.5 % ethandithiole (EDT). The mixture was allowed to stand at 25 °C for 2.5 hours and thereafter concentrated under vacuum. After precipitation of the cyclic fully deprotected peptide in diethylether ($Et_2O$) at 0°C the solid was washed twice with $Et_2O$ and dried. Cyclic peptides without designed beta-strand linkages were purified by reverse phase HPLC, cyclic peptides arranged for additional beta-strand linkages were processed as described below.

*Formation of disulfide β-strand linkage and purification*

After deprotection, the crude peptide was dissolved in 9 ml of 5% AcOH (buffered with $NaHCO_3$ to pH 5-6). 0.5 ml DMSO were added and the solution was shaken overnight. Following evaporation the residue was purified by preparative reverse phase HPLC.

Analytical method 1a:

Analytical HPLC retention times (RT, in minutes) were determined using an Acquity UPLC BEH C18 1.7 μm column with the following solvents A ($H_2O/CH_3CN$, 95/5 [v/v], + 0.1 % TFA) and B ($CH_3CN$ + 0.09 % TFA) and the gradient: 0 min: 99 % A, 1 % B; 0.2 min: 99 % A, 1 % B; 4 min: 5 % A, 95 % B; 4.2 min: 5 % A, 95 % B; 4.25 min: 99 % A, 1 % B; 5.0 min: 99 % A, 1 % B.

Analytical method 1b:

Analytical HPLC retention times (RT, in minutes) were determined using an Acquity UPLC BEH C18 1.7 μm column with the following solvents A ($H_2O$ +0.1 % TFA) and B ($CH_3CN$ + 0.09 % TFA) and the gradient: 0 min: 95 % A, 5 % B; 0.2 min: 95 % A, 5 % B; 4 min: 5 % A, 95 % B; 4.2 min: 5 % A, 95 % B; 4.25 min: 95 % A, 5 % B; 5.0 min: 95 % A, 5 % B.

Analytical method 2:

Analytical HPLC retention times (RT, in minutes) were determined using an Acquity UPLC BEH C18 1.7 μm column with the following solvents A ($H_2O/CH_3CN$, 95/5 [v/v], + 0.1 % TFA) and B ($CH_3CN$ + 0.09 % TFA) and the gradient: 0 min: 99 % A, 1 % B; 0.2 min: 99 % A, 1 % B; 4 min: 35 % A, 65 % B; 4.05 min: 5 % A, 95 % B; 4.20 min: 5 % A, 95 % B; 4.25 min: 99 % A, 1 % B; 4.5 min: 99 % A, 1 % B.

Analytical method 3:

Analytical HPLC retention times (RT, in minutes) were determined using a zorbaxEclipsedXDBC18 column with the following solvents A ($H_2O$ + 0.1 % TFA) and B ($CH_3CN$ + 0.1 % TFA) and the gradient: 0 min: 60 % A, 40 % B; 21 min: 10 % A, 90 % B; 27 min: 100 % B.

Analytical method 4:

Analytical HPLC retention times (RT, in minutes) were determined using a Laubscher Labs Interchrom 218QTP54 C18, 250 x 4.6 mm, 5 μm, 300 A with the following solvents A ($H_2O$ + 0.1 % TFA) and B ($CH_3CN$ + 0.1 % TFA) and the gradient: 0 min: 70 % A, 30 % B; 16.7 min: 100 % B.

**Example 28** is shown in *Table 1.* The peptide was synthesized starting with the amino acid Pro which was grafted to the resin. Starting resin was Fmoc-ProO-chlorotrityl resin, which was prepared as described above. The linear peptide was synthesized on solid support according to the procedure described above in the following sequence: Resin-Pro-$^D$Ser-P8-P7-P6-P5-P4-P3-P2-P1. Following a final Fmoc deprotection as described above, the peptide was cleaved from the resin, cyclized, deprotected and after formation of the disulfide beta-strand linkage purified as indicated above. HPLC-retention time (minutes) was determined using the gradient method 2 as described above

**Example 29** is shown in *Table 1.* The peptide was synthesized starting with the amino acid Pro which was grafted to the resin. Starting resin was Fmoc-ProO-chlorotrityl resin, which was prepared as described above. The linear peptide was synthesized on solid support according to the procedure described above in the following sequence: Resin-Pro-$^D$Hyp-P8-P7-P6-P5-P4-P3-P2-P1. Following a final Fmoc deprotection as described above, the peptide was cleaved from the resin, cyclized, deprotected and after formation of the disulfide beta-strand linkage purified as indicated above. HPLC-retention time (minutes) was determined using the gradient method 2 as described above

**Example 30** is shown in *Table 1.* The peptide was synthesized starting with the amino acid Pro which was grafted to the resin. Starting resin was Fmoc-ProO-chlorotrityl resin, which was prepared as described above. The linear peptide was synthesized on solid support according to the procedure described above in the following sequence: Resin-Pro-$^D$Glu-P8-P7-P6-P5-P4-P3-P2-P1. Following a final Fmoc deprotection as described above, the peptide was cleaved from the resin, cyclized, deprotected and after formation of the disulfide beta-strand linkage purified as indicated above. HPLC-retention time (minutes) was determined using the gradient method 2 as described above

**Examples 31-35** are shown in *Table 1*. The peptides were synthesized starting with the amino acid Pro which was grafted to the resin. Starting resin was Fmoc-ProO-chlorotrityl resin, which was prepared as described above. The linear peptides were synthesized on solid support according to the procedure described above in the following sequence: Resin-Pro-$^D$Pro-P8-P7-P6-P5-P4-P3-P2-P1. Following a final Fmoc deprotection as described above, the peptides were cleaved from the resin, cyclized, deprotected and after formation of the disulfide beta-strand linkage purified as indicated above.
HPLC-retention times (minutes) were determined using the gradient method 2 as described above

Table 1: Examples (Ex.)

| Ex. | Seq ID | P1 | P2 | P3 | P4 | P5 | P6 | P7 | P8 | Template | Purity %[a)] | [M+ H] | RT |
|---|---|---|---|---|---|---|---|---|---|---|---|---|---|
| 28 | SEQ ID NO: 28 | Asp | Cys | Phe | Trp | Lys | Tyr | Cys | Leu | $^D$Ser$^L$Pro | 95 | 1243.0 | 3.12 |
| 29 | SEQ ID NO: 29 | Asp | Cys | Phe | Trp | Lys | Tyr | Cys | Leu | $^D$4Hyp2$^L$Pro | 95 | 1267.8 | 3.05 |
| 30 | SEQ ID NO: 30 | Asp | Cys | Phe | Trp | Lys | Tyr | Cys | Leu | $^D$Glu$^L$Pro | 90 | 1283.5 | 3.10 |
| 31 | SEQ ID NO: 31 | Asp | Cys | Phe | Trp | Lys | Tyr | Cys | Val | $^D$Pro$^L$Pro | 90 | 1237.3 | 3.11 |
| 32 | SEQ ID NO: 32 | Asp | Cys | Phe | $^D$Trp | Orn | Tyr | Cys | Val | $^D$Pro$^L$Pro | 90 | 1223.4 | 3.06 |
| 33 | SEQ ID NO: 33 | Asp | Cys | Tyr | Trp | Lys | Tyr | Cys | Leu | $^D$Pro$^L$Pro | 90 | 1267.5 | 2.95 |
| 34 | SEQ ID NO: 34 | Asp | Cys | Phe | $^D$Trp | Lys | Tyr | Cys | Val | $^D$Pro$^L$Pro | 85 | 1237.5 | 3.07 |
| 35 | SEQ ID NO: 35 | Asp | Cys | Phe | Trp | Lys | Tyr | Cys | Cha | $^D$Pro$^L$Pro | 90 | 1292.8 | 3.60 |

Cys in pos.2 and 7 in Ex. 28-35 form a disulfide bridge,
a) %-purity of compounds after prep. HPLC.

### 2. Biological methods

### 2.1. Preparation of the peptides

Lyophilized peptides were weighed on a Microbalance (Mettler MT5) and dissolved in sterile water to a final concentration of 1 mM unless stated otherwise. Stock solutions were kept at +4°C, light protected.

### 2.2. Cell culture

Mouse pre-B cells were cultured in RPMI1640 plus 5 % FBS, antibiotic/antimycotic, non essential amino acid, 50 μM beta-mercaptoethanol and 1mM natrium pyruvate. HELA cells were maintained in RPMI1640 plus 10 % FBS, pen/strept and 2 mM L-glutamine. Cos-7 cells were grown in DMEM medium with 4500 mg/mL glucose supplemented with 10 % FCS, pen/strept and 2 mM L-glutamine. All cell lines were grown at 37° C at 5 % $CO_2$. Cell media, media supplements, PBS-buffer, HEPES, antibiotic/antimycotic, pen/strept, non essential amino acid, L-glutamine, beta-mercaptoethanol and sera were purchased from Gibco (Pailsey, UK). All fine chemicals were supplied by Merck (Darmstadt, Germany).

### 2.3. $Ca^{2+}$-assay: UTR2 receptor-agonizing and antagonizing activity of the peptides

The mouse pre-B cell line 300-19 was stably transfected with the cDNA encoding the human UTR2 receptor (GenBank Acc# NM_018949), and expression was confirmed with a positive calcium signal in response to human urotensin (Sigma Aldrich). Increases in intracellular calcium were monitored using a Flexstation 384 (Molecular Devices, Sunnyvale, CA). The cells were batch loaded with the Calcium 4 Assay kit (Molecular Devices) in assay buffer (Hanks Balanced salt solution, HBSS, 20 mM HEPES, pH 7.4, 0.1% BSA) for 1 h at room temperature and labeled cells were dispensed into either black 96 well or 384 well assay plates (Greiner). Calcium mobilization induced by urotensin or test compounds was measured in the Flexstation 384 (excitation, 485 nm; emission, 525 nm) for 70 seconds. Agonist activity was determined by direct addition of ligand or peptides, while antagonists were identified by spiking the cells with test compounds prior to urotensin addition. A dose response curve (compound concentration versus % maximum response for urotensin) was determined for each active agonist and antagonist and was fitted to a four parameter logistic equation using SoftmaxPro 4.8 (Molecular Devices), from which EC50% and IC50% values were calculated.

Following normalization data were fitted with IGORpro software® (WaveMetrics, Lake Oswego, OR, USA) to a sigmoid equation to determine $IC_{50}$ values. The $K_i$ values were calculated from $IC_{50}$ values according to the method described by Nikolovska-Coleska et al. Anal. Biochem., 2004, 332, 261).

### 2.4. Cytotoxicity assay

The cytotoxicity of the peptides to HELA cells (Acc57) and COS-7 cells (CRL-1651) was determined using the MTT reduction assay. Briefly, the method was as follows: 7000 HELA cells/well and 4500 COS-7 cells/well were seeded and grown in 96-well microtiter plates for 24 h at 37 °C at 5 % $CO_2$. Thereafter, time zero (Tz) was determined by MTT reduction (see below). The supernatant of the remaining wells was discarded, and fresh medium and compounds in serial dilutions (12.5, 25 and 50 μM, triplicates) were pipetted into the wells. After incubation of the cells for 48 h at 37° C at 5 % $CO_2$ the supernatant was discarded again and 100 μL MTT reagent (0.5 mg/mL in RPMI1640 and DMEM, respectively)/well was added. Following incubation at 37° C for 2 h the media were aspirated and the cells were spiked (100 μl isopropanol/well). The absorbance of the solubilized formazan was measured at 595 nm ($OD_{595}$peptide). For each concentration averages were calculated from triplicates. The percentage of growth was calculated as follows: ($OD_{595}$peptide-$OD_{595}$Tz-$OD_{595}$Empty well)/($OD_{595}$Tz-$OD_{595}$Empty well) x 100 %. The $GI_{50}$ (Growth Inhibition) concentrations were calculated for each peptide by using a trend line function for the concentrations (50, 25, 12.5 and 0 μM), the corresponding percentages and the value 50, (=TREND ($C_{50}$:$C_0$,$\%_{50}$:$\%_0$,50).

### 2.5. Hemolysis

The peptides were tested for their hemolytic activity against human red blood cells (hRBC). Fresh hRBC were washed three times with phosphate buffered saline (PBS) and centrifuged for 10 min at 2000 x g. Compounds (100 μM) were incubated with 20% hRBC (v/v) for 1 h at 37° C. The final erythrocyte concentration was approximately 0.9 x $10^9$ cells/mL. A value of 0 % and 100 % cell lyses, respectively, was determined by incubation of hRBC in the presence of PBS alone and 0.1 % Triton X-100 in $H_2O$, respectively. The samples were centrifuged, the supernatants were 20-fold diluted in PBS buffer and the optical densities (OD) were measured at 540 nm. The 100 % lyses value ($OD_{540}H_2O$) gave an $OD_{540}$ of approximately 1.3-1.8. Percent hemolysis was calculated as follows:

$$(OD_{540}\text{peptide}/OD_{540}H_2O)\ \text{x}100\ \%.$$

### 2.6. Plasma stability

The stability of the peptides in human and mouse plasma was determined by applying the following method: 315 μl/deep well of freshly thawed human plasma (Basler Blutspendedienst) and mouse plasma (Harlan Sera-Lab, UK), respectively, were spiked with 35 μl/well of compound in PBS (100 μM, triplicate) and incubated at 37° C. At t = 0, 15, 30, 60, 120 and 240 min aliquots of 50 μl were transferred to filtration plate wells containing 150 μl/well of acetonitrile. Following shaking for 2 min the occurred suspensions were filtrated by vacuum and finally, 100 μl of each filtrate were transferred to a propylene microtiter plate, and analyzed by LC/MS as follows: Column: Waters, XBridge C18, mobile phases: (A) water + 0.1 % formic acid and (B) acetonitrile/water, 95/5 (v/v) + 0.1 % formic acid, gradient: 5%-100% (B) in 2 minutes, electrospray ionization, MRM detection (triple quadrupole) . The peak areas were determined and triplicate values were averaged. The stability was expressed in percent of the initial value at t = 0. ($t_x/t_0$ x 100). By using the TREND function of EXCEL (Microsoft Office 2003) $T_{1/2}$ were determined.

**Table 1**

| **Ex.** | **EC50% [nM], Urotensin II receptor** |
|---|---|
| **28** | <2 |
| **29** | <2 |
| **30** | <2 |
| **31** | <2 |
| **33** | <2 |
| **34** | <2 |

**Table 2**

| **Ex.** | **IC50% [nM] ±SD, Urotensin II receptor** |
|---|---|
| **32** | 0.03 ± 0.01 |
| **35** | 0.2 ± 0.01 |

**Table 3**

| **Ex.** | **Cytotoxicity** | | | **Plasmastability** | |
|---|---|---|---|---|---|
| | Hela Cells $GI_{50}$ [μM] | Cos-7 Cells $GI_{50}$ [μM] | **Hemolysis** at 100 μM [%] | human pl. $T_{1/2}$ [min] | mouse pl. $T_{1/2}$ [min] |
| **28** | > 50 | > 50 | 0 | 240 | 240 |
| **29** | > 50 | > 50 | 0 | 240 | 240 |
| **30** | > 50 | > 50 | 0 | 240 | 240 |
| **31** | > 50 | > 50 | 0 | 240 | 240 |
| **32** | > 50 | > 50 | 0 | 240 | 240 |
| **33** | > 50 | > 50 | 0 | 240 | 240 |
| **34** | > 50 | > 50 | 0 | 240 | 240 |
| **35** | > 50 | > 50 | 0 | 240 | 240 |

### Claims

**1.** Compounds of the general formula

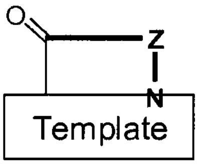

(I)

wherein

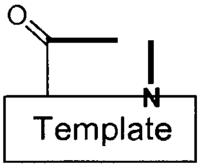

is $^{D}$Pro-$^{L}$Pro, $^{D}$Ser-LPro or $^{D}$Glu-$^{L}$Pro and Z is a chain of 8 alpha-amino acid residues, the positions of said amino acid residues in said chain being counted starting from the N-terminal amino acid, whereby these amino acid residues are, depending on their position in the chain,

- P1: Asp;
- P2: Cys;
- P3: Phe, Tyr;
- P4: Trp, $^{D}$Trp;
- P5: Lys, Orn;
- P6: Tyr;
- P7: Cys,
- P8: Cha, Leu, Val; and
- Cys at P2 and P7 can form a disulfide bridge,

and pharmaceutically acceptable salts thereof.

**2.** A compound of formula I according to claim 1 wherein the template is $^{D}$Pro-$^{L}$Pro and the amino acid residues in position 1 - 8 are:

- P1: Asp;
- P2: Cys;
- P3: Phe;
- P4: Trp;
- P5: Lys;
- P6: Tyr;
- P7: Cys; and
- P8: Val;

Cys at P2 and P7 forming a disulfide bridge.

**3.** A compound of formula I according to claim 1 wherein the template is $^{D}$Pro-$^{L}$Pro and the amino acid residues in position 1-8 are:

- P1: Asp;
- P2: Cys;
- P3: Phe;
- P4: $^{D}$Trp;
- P5: Orn;
- P6: Tyr;

- P7: Cys; and
- P8: Val;

Cys at P2 and P7 forming a disulfide bridge.

**4.** Enantiomers of the compounds of formula I as defined in claim 1.

**5.** Compounds according to any one of claims 1 to 4 for use as therapeutically active substances.

**6.** Compounds according to claim 5 having agonizing or antagonizing activity against urotensin II being useful for treating renal disease, diabetes, cardiovascular dysfunction, inflammation as well as allergic airways diseases like allergic rhinitis and asthma.

**7.** A pharmaceutical composition containing a compound according to any one of claims 1 to 4 and a pharmaceutically inert carrier.

**8.** Compositions according to claim 7 in a form suitable for oral, topical, transdermal, injection, buccal, transmucosal, pulmonary or inhalation administration.

**9.** Compositions according to claim 7 or 8 in form of tablets, dragees, capsules, solutions, liquids, gels, plaster, creams, ointments, syrup, slurries, suspensions, spray, nebuliser or suppositories.

**10.** The use of compounds according to any one of claims 1 to 4 for the manufacture of a medicament for use as an agonist or antagonist of urotensin II.

**11.** The use according to claim 10 wherein said urotensin II agonizing medicament is intended to be used in cases where renal disease is mediated or resulting from, or where diabetes is mediated or resulting from, or where cardiovascular dysfunction is mediated or resulting from, or where inflammation is mediated or resulting from urotensin II activity.

**12.** A process for the manufacture of compounds according to any one of claims 1-3 which process comprises

(a) coupling an appropriately functionalized solid support with an appropriately N-protected derivative of that amino acid which in the desired end-product is in positions 3, 4 or 5, any functional group which may be present in said N-protected amino acid derivative being likewise appropriately protected;
(b) removing the N-protecting group from the product thus obtained;
(c) coupling the product thus obtained with an appropriately N-protected derivative of that amino acid which in the desired end-product is one position nearer the N-terminal amino acid residue, any functional group which may be present in said N-protected amino acid derivative being likewise appropriately protected;
(d) removing the N-protecting group from the product thus obtained;
(e) repeating steps (c) and (d) until the N-terminal amino acid residue has been introduced;
(f) coupling the product thus obtained with a compound of the general formula

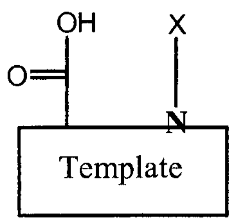

**II**

wherein

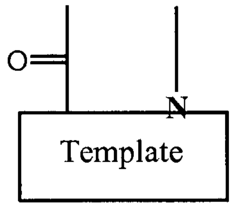

is as defined in claim 1 and X is an N-protecting group or, alternatively,

(fa) coupling the product obtained in step (e) with an appropriately N-protected derivative of an amino acid of the general formula

HOOC-B-H **III**

or

HOOC-A-H **IV**

wherein B and A are as defined in claim 1, any functional group which may be present in said N-protected amino acid derivative being likewise appropriately protected;
(fb) removing the N-protecting group from the product thus obtained; and
(fc) coupling the product thus obtained with an appropriately N-protected derivative of an amino acid of the above general formula IV or formula

HOOC-B3-H **V**

wherein B3 is as defined in claim 1

and, respectively, formula III, any functional group which may be present in said N-protected amino acid derivative being likewise appropriately protected;
(g) removing the N-protecting group from the product obtained in step (f) or (fc);
(h) coupling the product thus obtained with an appropriately N-protected derivative of that amino acid which in the desired end-product is in position, 8 any functional group which may be present in said N-protected amino acid derivative being likewise appropriately protected;
(i) removing the N-protecting group from the product thus obtained;
(j) coupling the product thus obtained with an appropriately N-protected derivative of that amino acid which in the desired end-product is one position farther away from position 8, any functional group which may be present in said N-protected amino acid derivative being likewise appropriately protected;
(k) removing the N-protecting group from the product thus obtained;
(l) repeating steps (j) and (k) until all amino acid residues have been introduced;
(m) if desired, selectively deprotecting one or several protected functional group(s) present in the molecule and appropriately substituting the reactive group(s) thus liberated;
(n) if desired, forming an interstrand linkage between side-chains of appropriate amino acid residues at positions 2 and 7;
(o) detaching the product thus obtained from the solid support;
(p) cyclizing the product cleaved from the solid support;
(q) removing any protecting groups present on functional groups of any members of the chain of amino acid residues and, if desired, any protecting group(s) which may in addition be present in the molecule; and
(r) if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

**13.** A process for the manufacture of compounds according to any one of claims 1-3 which process comprises

(a') coupling an appropriately functionalized solid support with a compound of the general formula

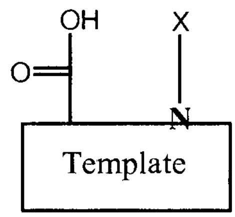

II

wherein

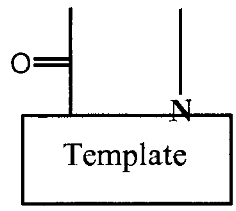

is as defined in claim 1 and X is an N-protecting group or, alternatively,

(a'a) coupling said appropriately functionalized solid support with an appropriately N-protected derivative of an amino acid of the general formula

HOOC-B-H **III**

or

HOOC-A-H **IV**

wherein B and A are as defined in claim 1, any functional group which may be present in said N-protected amino acid derivative being likewise appropriately protected;
(a'b) removing the N-protecting group from the product thus obtained; and
(a'c) coupling the product thus obtained with an appropriately N-protected derivative of an amino acid of the above general formula IV or formula

HOOC-B3-H **V**

wherein B3 is as defined in claim 1

and, respectively, formula III, any functional group which may be present in said N-protected amino acid derivative being likewise appropriately protected;
(b') removing the N-protecting group from the product obtained in step (a') or (a'c) (c') coupling the product thus obtained with an appropriately N-protected derivative of that amino acid which in the desired end-product is in position 8, any functional group which may be present in said N-protected amino acid derivative being likewise appropriately protected;
(d') removing the N-protecting group from the product thus obtained;
(e') coupling the product thus obtained with an appropriately N-protected derivative of that amino acid which in the desired end-product is one position farther away from position 8, any functional group which may be present in said N-protected amino acid derivative being likewise appropriately protected;
(f') removing the N-protecting group from the product thus obtained;
(g') repeating steps (e') and (f') until all amino acid residues have been introduced;
(h') if desired, selectively deprotecting one or several protected functional group(s) present in the molecule and appropriately substituting the reactive group(s) thus liberated;
(i') if desired forming an interstrand linkage between side-chains of appropriate amino acid residues at opposite positions 2 and 7;

(j') detaching the product thus obtained from the solid support;

(k') cyclizing the product cleaved from the solid support;

(l') removing any protecting groups present on functional groups of any members of the chain of amino acid residues and, if desired, any protecting group(s) which may in addition be present in the molecule; and

(m') if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

**14.** A process of claim 12 or 13, for the manufacture of compounds according to claim 4, modified in that enantiomers of all starting materials are used.

**Patentansprüche**

**1.** Verbindungen mit der allgemeinen Formel

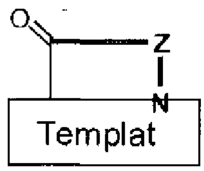

**(I)**

wobei

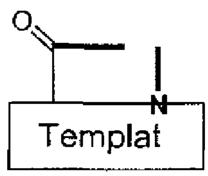

$^{D}$Pro-$^{L}$Pro, $^{D}$Ser-$^{L}$Pro oder $^{D}$Glu-$^{L}$Pro ist und Z eine Kette von 8 alpha-Aminosäureresten ist, wobei die Positionen der Aminosäurereste in der Kette beginnend an der N-terminalen Aminosäure gezählt werden, wobei diese Aminosäurereste in Abhängigkeit von ihrer Position in der Kette wie folgt sind:

P1: Asp;
P2: Cys;
P3: Phe, Tyr;
P4: Trp, $^{D}$Trp;
P5: Lys, Orn;
P6: Tyr;
P7: Cys,
P8: Cha, Leu, Val; und
Cys an P2 und P7 eine Disulfidbrücke bilden können,

und pharmazeutisch annehmbare Salze davon.

**2.** Verbindung der Formel I nach Anspruch 1, wobei das Templat $^{D}$Pro-$^{L}$Pro ist und die Aminosäurereste in Position 1-8 wie folgt sind:

P1: Asp;
P2: Cys;
P3: Phe;
P4: Trp;

P5: Lys;
P6: Tyr;
P7: Cys und
P8: Val;
Cys an P2 und P7 eine Disulfidbrücke bilden.

**3.** Verbindung der Formel I nach Anspruch 1, wobei das Templat $^{D}$Pro-$^{L}$Pro ist und die Aminosäurereste in Position 1-8 wie folgt sind:

P1: Asp;
P2: Cys;
P3: Phe;
P4: $^{D}$Trp;
P5: Orn;
P6: Tyr;
P7: Cys und
P8: Val;
Cys an P2 und P7 eine Disulfidbrücke bilden.

**4.** Enantiomere der Verbindungen der Formel I wie in Anspruch 1 definiert.

**5.** Verbindungen nach einem der Ansprüche 1 bis 4 zur Verwendung als therapeutisch aktive Substanzen.

**6.** Verbindungen nach Anspruch 5 mit agonisierender oder antagonisierender Aktivität gegen Urotensin II, die zur Behandlung von Nierenerkrankung, Diabetes, kardiovaskulärer Dysfunktion, Entzündung sowie allergischen Erkrankungen der Atemwege, wie allergischer Rhinitis und Asthma, brauchbar sind.

**7.** Pharmazeutische Zusammensetzung, die eine Verbindung nach einem der Ansprüche 1 bis 4 und einen pharmazeutisch inerten Träger enthält.

**8.** Zusammensetzungen nach Anspruch 7 in einer Form, die zur oralen, topischen, transdermalen, Injektions-, bukkalen, transmukosalen, pulmonaren oder Inhalationsverabreichung geeignet ist.

**9.** Zusammensetzungen nach Anspruch 7 oder 8 in Form von Tabletten, Dragees, Kapseln, Lösungen, Flüssigkeiten, Gels, Pflastern, Cremes, Salben, Sirup, Aufschlämmungen, Suspensionen, Spray, Vernebler oder Zäpfchen.

**10.** Verwendung von Verbindungen nach einem der Ansprüche 1 bis 4 zur Herstellung eines Medikaments zur Verwendung als Agonist oder Antagonist von Urotensin II.

**11.** Verwendung nach Anspruch 10, wobei das Urotensin II agonisierende Medikament in Fällen zu verwenden ist, bei denen Nierenerkrankung vermittelt wird durch Urotensin II-Aktivität oder daraus resultiert, oder bei denen Diabetes vermittelt wird durch Urotensin II-Aktivität oder daraus resultiert, oder bei denen kardiovaskuläre Dysfunktion vermittelt wird durch Urotensin II-Aktivität oder daraus resultiert, oder bei denen Entzündung vermittelt wird durch Urotensin II-Aktivität oder daraus resultiert.

**12.** Verfahren zur Herstellung von Verbindungen nach einem der Ansprüche 1-3, wobei das Verfahren umfasst:

(a) Koppeln eines geeignet funktionalisierten festen Trägers mit einem geeignet N-geschützten Derivat jener Aminosäure, die in dem gewünschten Endprodukt in den Positionen 3, 4 oder 5 ist, wobei jegliche funktionale Gruppe, die in dem N-geschützten Aminosäurederivat vorhanden sein kann, gleichermaßen geeignet geschützt ist;
(b) Entfernen der N-Schutzgruppe von dem so erhaltenen Produkt;
(c) Koppeln des so erhaltenen Produkts mit einem geeigneten N-geschützten Derivat jener Aminosäure, die in dem gewünschten Endprodukt eine Position näher an dem N-terminalen Aminosäurerest ist, wobei jegliche funktionale Gruppe, die in dem N-geschützten Aminosäurederivat vorhanden sein kann, gleichermaßen geeignet geschützt ist;
(d) Entfernen der N-Schutzgruppe von dem so erhaltenen Produkt;
(e) Wiederholen der Schritte (c) und (d), bis der N-terminale Aminosäurerest eingeführt worden ist;

(f) Koppeln des so erhaltenen Produkts mit einer Verbindung der allgemeinen Formel:

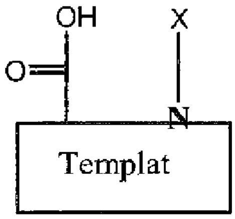

II

wobei

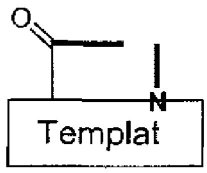

wie in Anspruch 1 definiert ist und X eine N-Schutzgruppe ist, oder alternativ

(fa) Koppeln des in Schritt (e) erhaltenen Produkts mit einem geeignet N-geschützten Derivat einer Aminosäure der allgemeinen Formel:

HOOC-B-H **III**

oder

HOOC-A-H **IV**

wobei B und A wie in Anspruch 1 sind, jegliche funktionale Gruppe, die in dem N-geschützten Aminosäurederivat vorhanden sein kann, gleichermaßen geeignet geschützt ist;
(fb) Entfernen der N-Schutzgruppe von dem so erhaltenen Produkt; und
(fc) Koppeln des so erhaltenen Produkts mit einem geeignet N-geschützten Derivat einer Aminosäure mit der obigen allgemeinen Formel IV oder Formel

HOOC-B3-H **V**

wobei B3 wie in Anspruch 1 definiert ist;
beziehungsweise Formel III, wobei jegliche funktionale Gruppe, die in dem N-geschützten Aminosäurederivat vorhanden sein kann, gleichermaßen geeignet geschützt ist;

(g) Entfernen der N-Schutzgruppe von dem in Schritt (f) oder (fc) erhaltenen Produkt;
(h) Koppeln des so erhaltenen Produkts mit einem geeigneten N-geschützten Derivat jener Aminosäure, die in dem gewünschten Endprodukt in Position 8 ist, wobei jegliche funktionale Gruppe, die in dem N-geschützten Aminosäurederivat vorhanden sein kann, gleichermaßen geeignet geschützt ist;
(i) Entfernen der N-Schutzgruppe von dem so erhaltenen Produkt;
(j) Koppeln des so erhaltenen Produkts mit einem geeigneten N-geschützten Derivat jener Aminosäure, die in dem gewünschten Endprodukt eine Position weiter entfernt von Position 8 ist, wobei jegliche funktionale Gruppe, die in dem N-geschützten Aminosäurederivat vorhanden sein kann, gleichermaßen geeignet geschützt ist;
(k) Entfernen der N-Schutzgruppe von dem so erhaltenen Produkt;
(l) Wiederholen der Schritte (j) und (k), bis alle Aminosäurereste eingeführt worden sind;
(m) gewünschtenfalls selektives Entschützen von einer oder mehreren geschützten funktionalen Gruppe(n), die in dem Molekül vorhanden sind, und geeignetes Substituieren der so freigesetzten reaktiven Gruppe(n);
(n) gewünschtenfalls Bilden einer Interstrangverknüpfung zwischen Seitenketten geeigneter Aminosäurereste

an den Positionen 2 und 7;
(o) Ablösen des so erhaltenen Produkts von dem festen Träger;
(p) Cyclisieren des von dem festen Träger abgespaltenen Produkts;
(q) Entfernen jeglicher Schutzgruppen, die an funktionalen Gruppen jeglicher Mitglieder der Kette der Aminosäurereste vorhanden sind, sowie gewünschtenfalls jeglicher Schutzgruppe/jeglichen Schutzgruppen, die zusätzlich in dem Molekül vorhanden sein können; und
(r) gewünschtenfalls Konvertieren des so erhaltenen Produkts in ein pharmazeutisch annehmbares Salz oder Konvertieren eines so erhaltenen pharmazeutisch annehmbaren oder unannehmbaren Salzes in die entsprechende freie Verbindung der Formel I oder in ein anderes pharmazeutisch annehmbares Salz.

**13.** Verfahren zur Herstellung von Verbindungen nach einem der Ansprüche 1-3, wobei das Verfahren umfasst:

(a') Koppeln eines geeignet funktionalisierten festen Trägers mit einer Verbindung der allgemeinen Formel

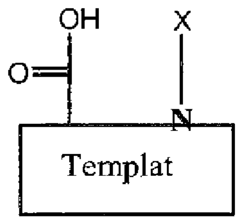

**II**

wobei

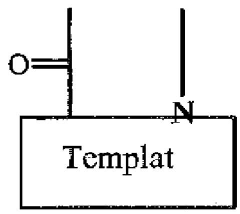

wie in Anspruch 1 definiert ist und X eine N-Schutzgruppe ist, oder alternativ (a'a) Koppeln des geeignet funktionalisierten festen Trägers mit einem geeignet N-geschützten Derivat einer Aminosäure mit der allgemeinen Formel

HOOC-B-H **III**

oder

HOOC-A-H **IV**

wobei B und A wie in Anspruch 1 sind, jegliche funktionale Gruppe, die in dem N-geschützten Aminosäurederivat vorhanden sein kann, gleichermaßen geeignet geschützt ist;
(a'b) Entfernen der N-Schutzgruppe von dem so erhaltenen Produkt; und
(a'c) Koppeln des so erhaltenen Produkts mit einem geeignet N-geschützten Derivat einer Aminosäure mit der obigen allgemeinen Formel IV oder Formel

HOOC-B3-H **V**

wobei B3 wie in Anspruch 1 definiert ist;
beziehungsweise Formel III, wobei jegliche funktionale Gruppe, die in dem N-geschützten Aminosäurederivat vorhanden sein kann, gleichermaßen geeignet geschützt ist;
(b') Entfernen der N-Schutzgruppe von dem in Schritt (a') oder (a'c) erhaltenen Produkt;

(c') Koppeln des so erhaltenen Produkts mit einem geeigneten N-geschützten Derivat jener Aminosäure, die in dem gewünschten Endprodukt in Position 8 ist, wobei jegliche funktionale Gruppe, die in dem N-geschützten Aminosäurederivat vorhanden sein kann, gleichermaßen geeignet geschützt ist;
(d') Entfernen der N-Schutzgruppe von dem so erhaltenen Produkt;
(e') Koppeln des so erhaltenen Produkts mit einem geeigneten N-geschützten Derivat jener Aminosäure, die in dem gewünschten Endprodukt eine Position weiter entfernt von Position 8 ist, wobei jegliche funktionale Gruppe, die in dem N-geschützten Aminosäurederivat vorhanden sein kann, gleichermaßen geeignet geschützt ist;
(f') Entfernen der N-Schutzgruppe von dem so erhaltenen Produkt;
(g') Wiederholen der Schritte (e') und (f'), bis alle Aminosäurereste eingeführt worden sind;
(h') gewünschtenfalls selektives Entschützen von einer oder mehreren geschützten funktionalen Gruppe(n), die in dem Molekül vorhanden sind, und geeignetes Substituieren der so freigesetzten reaktiven Gruppe(n);
(i') gewünschtenfalls Bilden einer Interstrangverknüpfung zwischen geeigneten Aminosäureresten an gegenüberliegenden Positionen 2 und 7;
(j') Ablösen des so erhaltenen Produkts von dem festen Träger;
(k') Cyclisieren des von dem festen Träger abgespaltenen Produkts;
(l') Entfernen jeglicher Schutzgruppen, die an funktionalen Gruppen jeglicher Mitglieder der Kette der Aminosäurereste vorhanden sind, sowie gewünschtenfalls jeglicher Schutzgruppe(n), die zusätzlich in dem Molekül vorhanden sein können; und
(m') gewünschtenfalls Konvertieren des so erhaltenen Produkts in ein pharmazeutisch annehmbares Salz oder Konvertieren eines so erhaltenen pharmazeutisch annehmbaren oder unannehmbaren Salzes in die entsprechende freie Verbindung der Formel I oder in ein anderes pharmazeutisch annehmbares Salz.

**14.** Verfahren nach Anspruch 12 oder 13 zur Herstellung von Verbindungen nach Anspruch 4, dahingehend modifiziert, dass Enantiomere aller Ausgangsmaterialien verwendet werden.

## Revendications

**1.** Composés de la formule générale

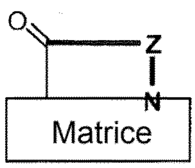

(I)

où

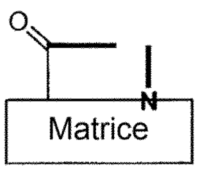

est $^{D}$Pro-$^{L}$Pro, $^{D}$Ser-$^{L}$Pro ou $^{D}$Glu-$^{L}$Pro et Z est une chaîne de 8 résidus d'acides alpha-aminés, les positions desdits résidus aminoacides dans ladite chaîne sont comptées à partir de l'acide aminé N-terminal, ces résidus aminoacides étant, en fonction de leur position dans la chaîne,

- P1 : Asp ;
- P2 : Cys ;
- P3 : Phe, Tyr ;
- P4 : Trp, $^{D}$Trp ;
- P5 : Lys, Orn ;

- P6 : Tyr;
- P7 : Cys ;
- P8 : Cha, Leu, Val ; et
- Cys en P2 et P7 pouvant former un pont disulfure,

et leurs sels pharmaceutiquement acceptables.

**2.** Composé de formule I selon la revendication 1, dans lequel la matrice est $^{D}$Pro-$^{L}$Pro et les résidus aminoacides aux positions 1 à 8 sont les suivants :

- P1 : Asp ;
- P2 : Cys ;
- P3 : Phe ;
- P4 : Trp ;
- P5 : Lys ;
- P6 : Tyr ;
- P7 : Cys ; et
- P8 : Val ;

Cys en P2 et P7 formant un pont disulfure.

**3.** Composé de formule I selon la revendication 1, dans lequel la matrice est $^{D}$Pro-$^{L}$Pro et les résidus aminoacides aux positions 1 à 8 sont les suivants :

- P1 : Asp ;
- P2 : Cys;
- P3 : Phe;
- P4 : $^{D}$Trp ;
- P5 : Orn ;
- P6 : Tyr;
- P7 : Cys ; et
- P8 : Val ;

Cys en P2 et P7 formant un pont disulfure.

**4.** Enantiomères des composés de formule I tels que définis dans la revendication 1.

**5.** Composés selon l'une quelconque des revendications 1 à 4 destinés à être utilisés comme substances thérapeutiquement actives.

**6.** Composés selon la revendication 5, ayant une activité agoniste ou antagoniste vis-à-vis de l'urotensine II et utiles pour traiter les maladies rénales, le diabète, les dysfonctionnements cardio-vasculaires, les inflammations ainsi que les maladies allergiques des voies respiratoires telles que la rhinite allergique et l'asthme.

**7.** Composition pharmaceutique contenant un composé selon l'une quelconque des revendications 1 à 4 et un support pharmaceutiquement inerte.

**8.** Compositions selon la revendication 7 se présentant sous une forme appropriée à l'administration orale, topique, transdermique, par injection, buccale, transmucosale, pulmonaire ou par inhalation.

**9.** Compositions selon la revendication 7 ou 8 se présentant sous la forme de comprimés, de dragées, de capsules, de solutions, de liquides, de gels, d'emplâtre, de crèmes, d'onguents, de sirops, de pâtes, de suspensions, de gouttelettes pulvérisées, de gouttelettes nébulisées ou de suppositoires.

**10.** Utilisation de composés selon l'une quelconque des revendications 1 à 4, dans la fabrication d'un médicament destiné à être utilisé comme agoniste ou antagoniste de l'urotensine II.

**11.** Utilisation selon la revendication 10, dans laquelle ledit médicament agoniste de l'urotensine II est destiné à être utilisé dans les cas où l'activité de l'urotensine II joue le rôle d'intermédiaire ou de déclencheur vis-à-vis de maladies rénales ou du diabète ou des dysfonctionnements cardiovasculaires ou d'inflammations.

**12.** Procédé de préparation de composés selon l'une quelconque des revendications 1 à 3, lequel procédé comprend

(a) le couplage d'un support solide convenablement fonctionnalisé à un dérivé, convenablement N-protégé, de cet acide aminé qui se trouve, dans le produit final souhaité, en position 3, 4 ou 5, tout groupe fonctionnel qui peut être présent dans ledit dérivé aminoacide N-protégé étant également protégé de manière appropriée ;
(b) l'élimination du groupe N-protecteur du produit ainsi obtenu ;
(c) le couplage du produit ainsi obtenu à un dérivé convenablement N-protégé de cet acide aminé qui se trouve, dans le produit final souhaité, à une position plus proche du résidu aminoacide N-terminal, tout groupe fonctionnel qui peut être présent dans ledit dérivé d'acide aminé N-protégé étant également protégé de manière appropriée ;
(d) l'élimination du groupe N-protecteur du produit ainsi obtenu ;
(e) la répétition des étapes (c) et (d) jusqu'à l'introduction du résidu aminoacide N-terminal ;
(f) le couplage du produit ainsi obtenu à un composé de la formule générale

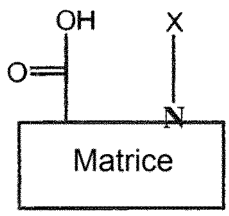

II

où

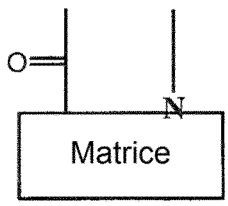

est tel que défini dans la revendication 1 et X est un groupe N-protecteur ou, en variante,

(fa) le couplage du produit obtenu à l'étape (e) à un dérivé convenablement N-protégé d'un acide aminé de la formule générale

HOOC-B-H III

ou

HOOC-A-H IV

B et A étant tels que définis dans la revendication 1, tout groupe fonctionnel qui peut être présent dans ledit dérivé aminoacide N-protégé étant également protégé de manière appropriée ;
(fb) l'élimination du groupe N-protecteur du produit ainsi obtenu ; et (fc) le couplage du produit ainsi obtenu à un dérivé convenablement N-protégé de l'acide aminé de la formule générale IV ci-dessus ou de la formule

HOOC-B3-H V

où B3 est tel que défini dans la revendication 1 et, respectivement, de la formule III, tout groupe fonctionnel qui peut être présent dans ledit dérivé aminoacide N-protégé étant également protégé de manière appropriée ;

(g) l'élimination du groupe N-protecteur du produit obtenu à l'étape (f) ou (fc) ;
(h) le couplage du produit ainsi obtenu à un dérivé convenablement N-protégé de cet acide aminé qui se trouve, dans le produit final souhaité, en position 8, tout groupe fonctionnel qui peut être présent dans ledit dérivé

aminoacide N-protégé étant également protégé de manière appropriée ;
(i) l'élimination du groupe N-protecteur du produit ainsi obtenu ;
(j) le couplage du produit ainsi obtenu à un dérivé convenablement N-protégé de cet acide aminé qui se trouve, dans le produit final souhaité, à une position plus éloignée de la position 8, tout groupe fonctionnel qui peut être présent dans ledit dérivé aminoacide N-protégé étant également protégé de manière appropriée ;
(k) l'élimination du groupe N-protecteur du produit ainsi obtenu ;
(l) la répétition des étapes (j) et (k) jusqu'à l'introduction de tous les résidus aminoacides ;
(m) si on le souhaite, la dé-protection sélective d'un ou de plusieurs groupes fonctionnels protégés présents dans la molécule et la substitution appropriée du ou des groupes réactifs ainsi libérés ;
(n) si on le souhaite, la formation d'une liaison inter-brins entre les chaînes latérales de résidus aminoacides appropriés aux positions 2 et 7 ;
(o) le détachement du produit ainsi obtenu du support solide ;
(p) la cyclisation du produit clivé du support solide ;
(q) l'élimination de tout groupe protecteur présent sur les groupes fonctionnels de tous membres de la chaîne de résidus aminoacides et, si on le souhaite, de tout groupe protecteur qui peut en outre être présent dans la molécule ; et
(r) si on le souhaite, la conversion du produit ainsi obtenu en un sel pharmaceutiquement acceptable ou la conversion d'un sel pharmaceutiquement acceptable ou inacceptable ainsi obtenu en le composé libre correspondant de formule I ou en un autre seul pharmaceutiquement acceptable.

**13.** Procédé de production de composés selon l'une quelconque des revendications 1 à 3, lequel procédé comprend

(a') le couplage d'un support solide convenablement fonctionnalisé à un composé de la formule générale

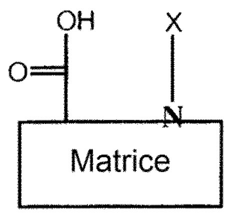

II

où

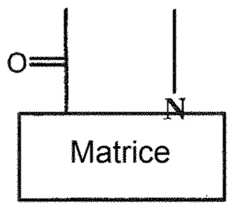

est tel que défini dans la revendication 1 et X est un groupe N-protecteur ou, en variante,

(a'a) le couplage dudit support solide convenablement fonctionnalisé à un dérivé convenablement N-protégé de l'acide aminé de la formule générale

HOOC-B-H III

ou

HOOC-A-H IV

B et A étant tels que définis dans la revendication 1, tout groupe fonctionnel qui peut être présent dans ledit dérivé aminoacide N-protégé étant également protégé de manière appropriée ;
(a'b) l'élimination du groupe N-protecteur du produit ainsi obtenu ; et

(a'c) le couplage du produit ainsi obtenu à un dérivé convenablement N-protégé de l'acide aminé de la formule générale IV ci-dessus ou de formule

HOOC-B3-H V

B3 étant tel que défini dans la revendication 1 et, respectivement, de formule III, toute groupe fonctionnel qui peut être présent dans ledit dérivé aminoacide N-protégé étant également protégé de manière appropriée ;

(b') l'élimination du groupe N-protecteur du produit obtenu à l'étape (a') ou (a'c) (c') le couplage du produit ainsi obtenu à un dérivé convenablement N-protégé de cet acide aminé qui se trouve, dans le produit final souhaité, en position 8, tout groupe fonctionnel qui peut être présent dans ledit dérivé aminoacide N-protégé étant également protégé de manière appropriée ;
(d') l'élimination du groupe N-protecteur du produit ainsi obtenu ;
(e') le couplage du produit ainsi obtenu à un dérivé convenablement N-protégé de cet acide aminé qui se trouve, dans le produit final souhaité, à une position plus éloignée de la position 8, tout groupe fonctionnel qui peut être présent dans ledit dérivé aminoacide N-protégé étant également protégé de manière appropriée ;
(f') l'élimination du groupe N-protecteur du produit ainsi obtenu ;
(g') la répétition les étapes (e') et (f') jusqu'à l'introduction de tous les résidus aminoacides ;
(h') si on le souhaite, la dé-protection sélective d'un ou de plusieurs groupes fonctionnels protégés présents dans la molécule et la substitution appropriée du ou des groupes réactifs ainsi libérés ;
(i') si on le souhaite, la formation d'une liaison inter-brins entre les chaînes latérales des résidus aminoacides appropriés aux positions opposées 2 et 7 ;
(j') le détachement du produit ainsi obtenu du support solide ;
(k') la cyclisation du produit clivé du support solide ;
(l') l'élimination de tout groupe protecteur présent sur les groupes fonctionnels de tous membres de la chaîne de résidus aminoacides et, si on le souhaite, de tout groupe protecteur qui peut en outre être présent dans la molécule ; et
(m') si on le souhaite, la conversion du produit ainsi obtenu en un sel pharmaceutiquement acceptable ou la conversion d'un sel pharmaceutiquement acceptable ou inacceptable ainsi obtenu en le composé libre correspondant de formule 1 ou en un autre sel pharmaceutiquement acceptable.

**14.** Procédé selon la revendication 12 ou 13, pour la préparation de composés selon la revendication 4, lequel procédé est modifié en ce que l'on utilise les énantiomères de toutes les matières de départ.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0232932 A
- EP 0201711 W
- WO 02070547 A1


### Non-patent literature cited in the description


- **TH. KLABUNDE ; G. HESSLER.** *ChemBioChem,* 2002, vol. 3, 928-44
- **A. WISE ; S.C. JUPE ; S. REES.** *Annu. Rev. Pharmacol. Toxicol.,* 2004, vol. 44, 43-66
- **T.K. ATTWOOD.** *Trends Pharmacol. Sci.,* 2001, vol. 22, 165-65
- **K. PALCZEWSKY et al.** *Science,* 2000, vol. 289, 739-45
- **M.C. GERSHENGORN et al.** *Endocrinology,* 2001, vol. 142, 2-10
- **S. SHACHAM et al.** *Med. Res. Rev.,* 2001, vol. 21, 472-83
- **S.G. RASMUSSEN et al.** *Nature,* 2007, vol. 450, 383-387
- **G. VAUQUELIN et al.** *Fundam. Clin. Pharmacol.,* 2005, vol. 19, 45-56
- **D. GHOSH ; A.G. PAPAVASSILIOU.** *Curr. Med. Chem.,* 2005, vol. 12, 691
- **C.M. HORVATH.** *TiBS,* 2000, vol. 25, 496
- **A.V. SCHALLY et al.** *Cell. Mol. Life Sci.,* 2004, vol. 61, 1042-68
- **W. ROSTÈNE et al.** *Encyclop. Biol. Chem.,* 2004, vol. 3, 3236
- **M. BOULES et al.** *Expert. Opin. Investig. Drugs,* 2005, vol. 14, 359-69
- **P. KITABGI.** *Curr. Opin. Drug Disc. Devel.,* 2002, vol. 5, 764-76
- **F. MARCEAU et al.** *Nat. Rev. Drug Disc.,* 2004, vol. 3, 845-52
- **M. ASHTON et al.** *Comb. Chem. And High Throughput Screening,* 2004, vol. 7, 441-53
- **E.R. SPINDEL et al.** *Recent Progress in Hormone Research,* 1993, vol. 48, 365-91
- **R.T. JENSEN et al.** Growth Factors, Peptides, and Receptors. Plenum Press, 1994, 225-237
- **G. ERTL et al.** *Drugs,* 2004, vol. 64, 1029-40
- **F.D. RUSSELL.** *Pharmacol. Ther.,* 2004, vol. 103, 223-43
- **A.V. SCHALLY et al.** *Cell. Mol. Life Sci.,* vol. 61, 1042-68
- **E. GHIO et al.** *Clin. Endocrinol.,* 2005, vol. 62, 1-17
- **B.G. IRANI et al.** *Curr. Pharm. Des.,* 2004, vol. 10, 3443-79
- **C.J. SMALL et al.** *Curr. Drug Targets CNS Neurol. Disord.,* vol. 3, 379-88
- **C.J. SMALL et al.** *Curr. Drug Targets CNS Neurol. Disord.,* 2004, vol. 3, 379-88
- **H.G. SELNICK et al.** *Curr. Med. Chem. Cardiovasc. Hematol. Agents,* 2003, vol. 1, 47-59
- **V.S. OSSOVSKAYA et al.** *Physiol. Rev.,* 2004, vol. 84, 579-621
- **A.M. COELHO et al.** *Curr. Med. Chem. Cardiovasc. Hematol. Agents,* 2003, vol. 1, 61-72
- **M. STEINHOFF et al.** *Endocrin. Rev.,* 2005, vol. 26, 1-43
- **A.L. KUNG ; S.D. ZABLUDOFF ; D.S. FRANCE et al.** *Cancer Cell,* 2004, vol. 6, 33
- **M. LEPOURCELET ; Y.N.P. CHEN ; D.S. FRANCE et al.** *Cancer Cell,* 2004, vol. 5, 91
- **T.R. GEISTLINGER ; R.K. GUY.** *J. Am. Chem. Soc.,* 2003, vol. 125, 6852
- **H. SHIMOGAWA ; Y. KWON ; Q. MAO et al.** *J. Am. Chem. Soc.,* 2004, vol. 126, 3461
- **D. OBRECHT ; M. ALTORFER ; J.A. ROBINSON.** *Adv. Med. Chem.,* 1999, vol. 4, 1-68
- **J.A. ROBINSON.** *Syn. Lett.,* 2000, vol. 4, 429-441
- **L. JIANG ; K. MOEHLE ; B. DHANAPAL ; D. OBRECHT ; J.A. ROBINSON.** *Helv. Chim. Acta,* 2000, vol. 83, 3097-3112
- **D. OBRECHT ; J.-M. VILLALGORDO.** Solid- Supported Combinatorial and Parallel Synthesis of Small-Molecular-Weight Compound Libraries. *Tetrahedron Organic Chemistry Series,* 1998, vol. 17
- **H. RINK.** *Tetrahedron Lett.,* 1987, vol. 28, 3783-3790
- **G.B. FIELDS ; C.G. FIELDS.** *J. Am. Chem. Soc.,* 1991, vol. 113, 4202-4207
- **MERGLER et al.** *Tetrahedron Lett.,* 1988, vol. 29, 4005-4008
- **H. RINK.** *Tetrahedron Lett.,* 1987, vol. 28, 3787-3790

- **FLÖRSHEIMER ; RINIKER.** *Peptides,* 1991, vol. 1990, 131
- **BARLOS et al.** *Tetrahedron Lett.,* 1989, vol. 30, 3943-3946
- **SHEEHAN ; HESS.** *J. Am. Chem. Soc.,* 1955, vol. 77, 1067-1068
- **SARANTAKIS et al.** *Biochem. Biophys. Res. Commun.,* 1976, vol. 73, 336-342
- **KÖNIG ; GEIGER.** *Chem. Ber,* 1970, vol. 103, 788-798
- **CASTRO et al.** *Tetrahedron Lett.,* 1975, vol. 14, 1219-1222
- *Synthesis,* 1976, 751-752
- **COSTE et al.** *Tetrahedron Lett.,* 1990, vol. 31, 205-208
- **KNORR et al.** *Tetrahedron Lett.,* 1989, vol. 30, 1927-1930
- **CARPINO et al.** *Tetrahedron Lett.,* 1994, vol. 35, 2279-2281
- **MARDER ; SHIVO ; ALBERICIO.** HCTU and TCTU: New Coupling Reagents: Development and Industrial Applications, Poster Presentation. *Gordon Conference,* February 2002
- **KAISER et al.** *Anal. Biochemistry,* 1970, vol. 34, 595
- **MEIENHOFER et al.** *Int. J. Peptide Protein Res.,* 1979, vol. 13, 35-42
- **FINGL et al.** The Pharmacological Basis of Therapeutics. 1975, 1
- **E. ATHERTON ; R. SHEPPARD.** Solid phase peptide synthesis. A practical approach. IRL Press, 1989, 49
- **NIKOLOVSKA-COLESKA et al.** *Anal. Biochem.,* 2004, vol. 332, 261